(19) Europäisches Patentamt
European Patent Office
Office européen des brevets

(11) Publication number: 0 191 399 B1

(12) EUROPEAN PATENT SPECIFICATION

(45) Date of publication of patent specification: 18.09.91

(51) Int. Cl.5: C07D 309/30, C07D 309/32, C07D 493/14, A61K 31/335, A61K 31/35, C12N 1/20, C12P 17/18, //(C07D493/14, 319:00,311:00,311:00), (C12N1/20,C12R1:465), (C12P17/18,C12R1:465)

(21) Application number: 86101412.4

(22) Date of filing: 04.02.86

# (54) Benz[a]anthraquinone compounds, a microbial process for their preparation and their use as medicaments.

(30) Priority: 09.02.85 JP 24085/85

(43) Date of publication of application:
20.08.86 Bulletin 86/34

(45) Publication of the grant of the patent:
18.09.91 Bulletin 91/38

(84) Designated Contracting States:
AT BE CH DE FR GB IT LI LU NL SE

(56) References cited:

CHEMICAL ABSTRACTS, vol. 97, no. 9, 30th August 1982, page 322, abstract no. 69008d, Columbus, Ohio, US; N. IMAMURA et al.: "Biosynthesis of vineomycins A1 and B2"

(73) Proprietor: MICROBIAL CHEMISTRY RESEARCH FOUNDATION
14-23, Kamiosaki 3 Chome Shinagawa-ku
Tokyo 141(JP)

(72) Inventor: Umezawa, Hamao, Prof.
23, Toyotama-kita 4-chome Nerima-ku
Tokyo(JP)
Inventor: Takeuchi, Tomio, No. 701A, New Fuji Mansion
1-11 Higashi-gotanda 5-chome Shinagawa-ku
Tokyo(JP)
Inventor: Sawa, Tsutomu
6-7, Ryosei 4-chome
Ayase-city Kanagawa Prefecture(JP)
Inventor: Hamada, Masa
No. 405, Shuwa Residence, 26, Naito-cho
1-chome
Shinjuku-ku Tokyo(JP)

EP 0 191 399 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

CHEMICAL ABSTRACTS, vol. 102, no. 23, 10th June 1985, page 623, abstract no. 204192y, Columbus, Ohio, US; K. OHTA et al.: "The absolute configuration of P-1894B, a potent prolyl hydroxylase inhibitor"

CHEMICAL ABSTRACTS, vol. 103, 23rd-30th December 1985, page 487, abstract no. 210513r, Columbus, Ohio, US; T. UCHIDA et al.: "Saquayamycins, new aquayamycin-group antibiotics"

Inventor: Naganawa, Hiroshi
17, Denenchofu-honcho 3-chome
Ota-ku Tokyo(JP)
Inventor: Uchida, Takeshi
22-18-102, Kishiya Tsurumi-ku
Yokohama-city Kanagawa Prefecture(JP)
Inventor: Imoto, Masaya
18-14-101, Tokiwa 7-chome
Urawa-city Saitama Prefecture(JP)

(74) Representative: Becker, Heinrich Karl Engelbert, Dr. et al
HOECHST AKTIENGESELLSCHAFT Central Patent Department P.O. Box 80 03 20
W-6230 Frankfurt am Main 80(DE)

## Description

Conventional benz[a]anthraquinone compounds obtained from microbial culture solutions include Tetrangomycin (Kuntsmann, M.P. et al: J. Org. Chem., 31, 2920-2925 (1966)), Ocromycin (Bowie, J.H. et al: Tetrahedron Lett., 1449-1452 (1967)), Aquayamycin (Sezaki, M. et al: Tetrahedron, 26, 5171-5190 (1970) and Sezaki, M. et al: J. Antibiotics, 21, 91-97 (1968)), Laveromycin (Lie, W-C. et al: J. Antibiotics, 23, 437-441 (1970)), SS-228Y (Kitahara, T. et al: J. Antibiotics, 28, 280-285 (1975)), Yoronomycin (Matsumura, et al: Japanese Patent Application No. 111554/1977) and Vineomycin A1 (Imamura, N. et al: Chem. Phar. Bull., 29, 1788-1790 (1981)). Antibiotics exist that have not been identified for chemical structure but which are believed to have the benz[a]anthraquinone skeleton in view of their absorption spectrum in the infrared and visible regions, and such antibiotics include Ayamycin A1, A2 and A3 (Satō, K. et al: J. Antibiotics, 13, 321-326 (1960)) and TA 435A (Nagahama, N. et al: J. Antibiotics Ser. B 17, 245-249 (1964)).

Many of the antibiotics listed above have been reported to have antitumor activities, but their activity levels are too low to encourage the carrying out of extensive research work with a view to developing them as cancer control agents.

Anthracycline antibiotics typified by Adriamycin form a family of cancer control agents used as key drugs in combined curing regimens of human cancers. It has however been pointed out recently that the advent of multi-drug resistant cells that exhibit resistance against a broad range of cancer control agents including Anthracycline lowers the therapeutical effects of anthracycline antibiotics and will eventually lead to the recurrence of the disease.

Benz[a]antrachinone antitumor antibiotics such as p 1894 B or vineomycin $A_1$ and $B_2$ are also described in Chem. Pharm. Bull. 1984, 32 (11), 4350-9 and J.Antibiot. 1982, 35 (5), 602-8.

The present inventors searched for benz[a]anthraquinone compounds that are effective against multi-drug resistant cells by screening compounds that have a skeletal structure very close to that of anthracycline antibiotics and which may exhibit high anti-tumor activities in vivo depending upon the side chains attached to the skeletal structure. As a result, it has been found that among the benz[a]anthraquinone antibiotics produced by actinomycetes, the compounds of formula (I) are as equally effective against Adriamycin-resistant P388 leukemia cells (hereunder abbreviated as P388/ADR) as against Adriamycin-sensitive P388 leukemia cells (hereunder abbreviated as P388/S). These compounds were equally effective in inhibiting the growth of P338/ADR and P388/S, and exhibited high activities in anti-tumor tests in vivo using mouse L/210 leukemia cells. Structural analyses of these compounds by various methods revealed that they are novel benz[a]anthraquinone compounds having the formula (I).

The benz[a]anthraquinone compounds (substances MH190) of the present invention are represented by the following formula (I):

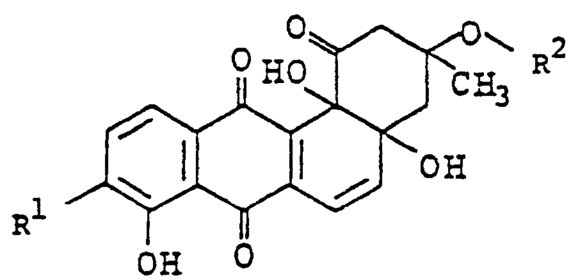

[I]

wherein $R^1$ and $R^2$ each represents one of the side chains listed in the following table:

Table 1

| Side chains as $R^1$ | | Side chain as $R^2$ | |
|---|---|---|---|
| II | 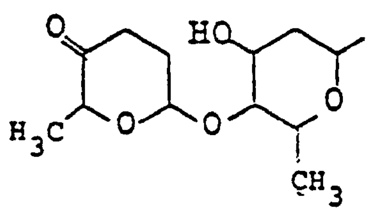 | V | 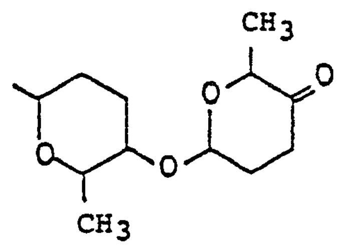 |
| III | 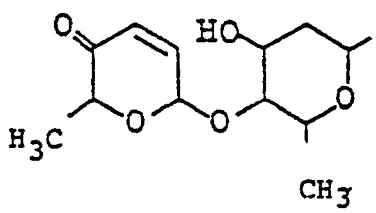 | VI | 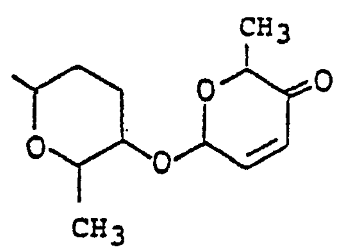 |
| IV | 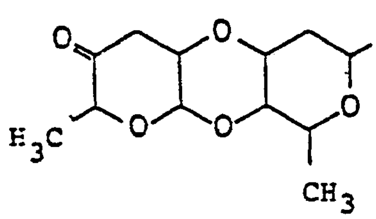 | | - H |

The present invention provides novel compounds falling within the category of benz[a]anthraquinone compounds, and it is quite surprising that these compound are effective against multi-drug resistant tumor cells.

The benz[a]anthraquinone compounds of the present invention have the chemical structure represented by formula (I) shown above.

In formula (I), $R^1$ and $R^2$ each represents one of the side chains (i) to (vi) listed in Table 1. Specific examples of the combination of these side chains are shown in Table 2 together with the symbols for the compounds having the respective combinations.

The compounds of the present invention are to be collectively named Saquayamycin. The symbols for the specific compounds are keyed to their names as shown below.

Table 2

| Symbol | $R^1$ | $R^2$ |
|---|---|---|
| MH190 Y1 | 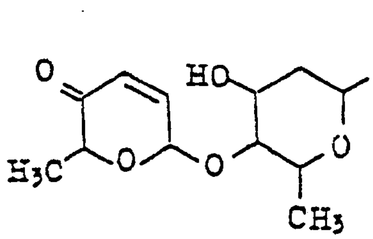 | 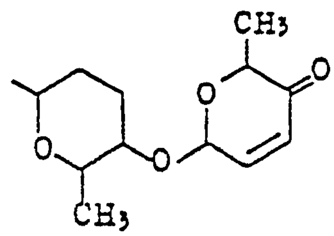 |
| MH190 Y2 | 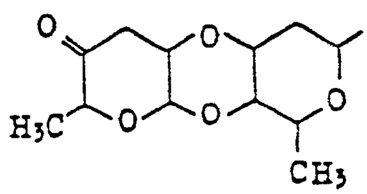 | 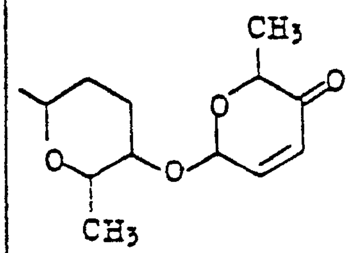 |
| MH190 Y3 | 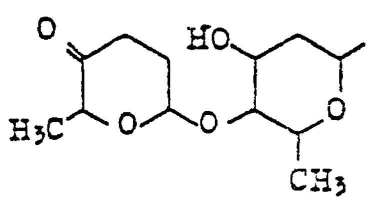 | 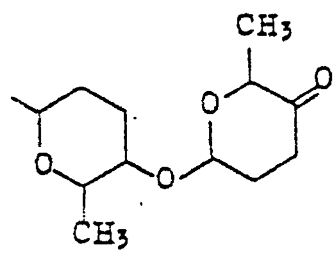 |
| MH190 Y4 | 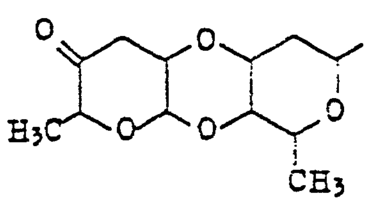 | 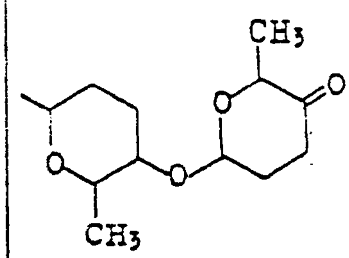 |

Table 2 (continued)

| Symbol | $R^1$ | $R^2$ |
|---|---|---|
| MH190 Y1-1 | 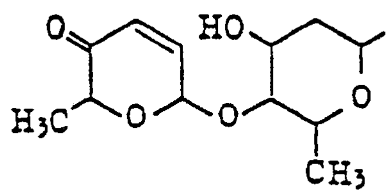 | - H |
| MH190 Y2-1 | 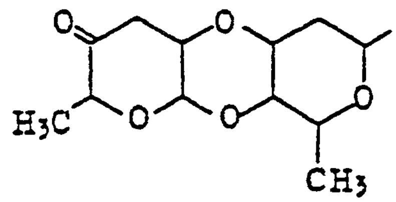 | - H |
| MH190 Y3-1 | 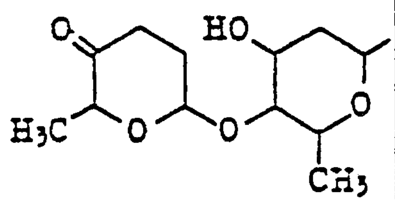 | - H |

| Symbol | Compound Name |
|---|---|
| MH190 | Saquayamycin |
| MH190 Y1 | Saquayamycin A |
| MH190 Y2 | Saquayamycin B |
| MH190 Y3 | Saquayamycin C |
| MH190 Y4 | Saquayamycin D |
| MH190 Y1-1 | Saquayamycin A1 |
| MH190 Y2-1 | Saquayamycin B1 |
| MH190 Y3-1 | Saquayamycin C1 |

The chemical structure of the compounds of the present invention was determined as follows:

Compounds MH190 Y1, Y2, Y3 and Y4 dissolved in a mixture of 0.4% aqueous phosphoric acid and acetonitrile (40:60) were left to stand overnight at room temperature, producing compounds MH190 Y1-1, Y2-1, Y3-1 and Y2-1, respectively.

When these compounds were heated in 0.1 - 0.5N HCℓ for periods of 15 - 30 minutes, each of them produced a yellow substance that showed Rf = 0.1 on silica gel TLC (chloroformmethanol (10:1)). This

substance was found to be Aquayamycin by comparison with an authentic sample of Aquayamycin (Sezaki, M. et al: Tetrahedron, 26, 5171-5190 (1970), and Sezaki M. et al: J. Antibiotics, 21, 91-97 (1968) in respect to Rf values on silica gel TLC, elution time in reverse-phase HPLC, absorption spectra in the UV and visible ranges, and specific rotation ($[\alpha]_D^{20}$ + 133°) (see Fig. 1).

Table 3 compares the $^{13}C$-NMR spectrum of Vineomycin AI, one of the Aquayamycin derivatives (Imamura, N. et al: J. Antibiotics, 35, 602-608 (1982)) with the $^{13}C$-NMR spectra of various types of substance MH190. Each of MH190 Y1, Y2, Y3, Y4, Y1-1 and Y2-1 is very close to the Aquayamycin derivative in terms of chemical shifts in the chromophore portion. This result shows that the substance MH190 is an Aquayamycin derivative.

MH190 Y1-1 provides $(M)^+$ at m/z 596 in FD-MS, gives a $^1H$-NMR spectrum having a coupling between 3'-proton and 3'-OH (see Table 4), and provides a $^{13}C$-NMR spectrum having a glycosidation shift at 4'-C (see Table 3). On the basis of these data, MH190 Y1-1 has been found to be a compound having acurose bonded to the Aquayamycin skeleton at 4'-position (see Fig. 1).

MH190 Y2-1 can also be obtained when MH190 Y1-1 is left on a silica gel, and it provides $(M)^+$ at m/z 596 in FD-MS, and gives $^1N$-NMR and $^{13}C$-NMR spectra which were respectively analyzed to be as shown in Tables 4 and 3. On the basis of these data, MH190 Y2-1 has been found to be a compound having cinerulose B bonded at 2- and 1-positions (2" and 1" in Fig. 1) respectively to 3'- and 4'-positions on the Aquayamycin skeleton.

MH190 Y3-1 forms as a result of catalytic reduction of MH190 Y1-1 catalyzed by Pd-$BaSO_4$ and provides a $^1H$-NMR spectrum analyzed as shown in Table 4. On the basis of these data, MH19O Y3-1 has been found to be a compound having cinerulose A bonded to the Aquayamycin skeleton at 4'-position (see Fig. 1).

When MH190 Y1 was treated with a mixture of 0.05N HCℓ and methanol at room temperature for 5 minutes, a spot that could be stained with $H_2SO_4$ formed on silica gel TLC a 2:1 mixture of benzene and ethyl acetate as a developing solvent. This spot agreed, in terms of Rf values on silica gel TLC, specific rotation ($[\alpha]_D^{20}$ - 20°), as well as EI-MS and $^1H$-NMR spectra, with the methyl disaccharide composed of rhodinose and acurose that was obtained by a similar treatment of Ditrisarbidin C (Uchida, T. et al: J. Antibiotics, 36, 1080-1083 (1983)). Analyses by $^1H$-NMR spectrum (Table 4) and $^{13}C$-NMR spectrum (Table 3) showed that MH190 Y1 is a compound having rhodinose and acurose bonded to MH190 Y1-1 at 3-position (Fig. 1).

MH190 Y2 can also be obtained when MH190 Y1 is left on silica gel, and it produces methyl disaccharide as in the case of MH190 Y1 by similar treatment with a mixture of 0.05N HCℓ and methanol, and provides $^1H$-NMR and $^{13}C$-NMR spectra as respectively shown in Tables 4 and 3. On the basis of these data, MH190 Y2 has been found to be a compound having rhodinose and acurose bonded to MH190 Y2-1 at 3-position.

When MH190 Y3 and Y4 were treated with a mixture of 0.05N HCℓ and methanol at room temperature for 5 minutes, both produced a spot on silica gel TLC at Rf = 0.41 (solvent system: benzene-ethyl acetate (2:1)) that could be stained with $H_2SO_4$. This spot was also produced by catalytic reduction of methyl disaccharide composed of rhodinose and acurose, and analyses by $^1H$-NMR and $^{13}C$-NMR spectra revealed that the spot was methyl disaccharide composed of rhodinose and cinerulose A. On the basis of these data, MH190 Y3 and Y4 have been determined to have structures wherein a disaccharide composed of rhodinose and cinerulose A are bonded at 3-position to MH190 Y3-1 and MH190 Y2-1. These structures are also supported by the $^1H$-NMR and $^{13}C$-NMR spectra of MH190 Y3 and Y4 respectively shown in Tables 4 and 3.

Table 3 Assignment of Chemical Shifts in $^{13}C$-NMR spectra (100 MHz, in d-$CHCl_3$)

| Carbon Assignment | M II 190 Y 1 | M II 190 Y 2 | M II 190 Y 3 | M II 190 Y 4 | M II 190 Y 1-1 | M II 190 Y 2-2 | Aquayamycin derivative |
|---|---|---|---|---|---|---|---|
| 1 | 204.7 | 204.7 | 204.7 | 204.7 | 204.8 | 204.7 | 203.6 |
| 2 | 50.2 | 50.2 | 50.2 | 50.2 | 53.1 | 53.1 | 48.0 |
| 3 | 82.4 | 82.4 | 82.4 | 82.4 | ( 76.0) | ( 76.0) | 78.8 |
| 4 | 44.5 | 44.5 | 44.5 | 44.5 | 43.3 | 43.3 | 43.3 |
| 4a | 79.9 | 79.9 | 79.9 | 79.9 | ( 80.5) | ( 80.5) | 81.2 |
| 5 | 145.6 | 145.6 | 145.6 | 145.6 | 144.3 | 144.3 | 144.4 |
| 6 | 117.4 | 117.4 | 117.5 | 117.4 | 117.5 | 117.5 | 116.2 |
| 6a | 138.8 | 138.8 | 138.8 | 138.8 | (138.2) | (138.0) | 137.3 |
| 7 | 188.2 | 188.3 | 188.2 | 188.3 | 188.0 | 188.0 | 187.1 |
| 7a | 114.0 | 114.0 | 114.0 | 114.0 | 114.0 | 114.0 | 112.9 |
| 8 | 158.0 | 157.9 | 158.1 | 157.9 | 158.1 | 158.0 | 156.8 |
| 9 | 138.2 | 137.8 | 138.4 | 137.8 | (138.4) | (138.2) | 137.6 |
| 10 | 133.5 | 133.7 | 133.6 | 133.7 | 133.7 | 133.8 | 132.5 |
| 11 | 119.6 | 119.6 | 119.6 | 119.6 | 119.8 | 119.8 | 118.4 |
| 11a | 130.5 | 130.6 | 130.5 | 130.6 | 130.4 | 130.4 | 129.3 |
| 12 | 182.2 | 182.2 | 182.2 | 182.2 | 182.0 | 182.0 | 181.0 |
| 12a | 138.9 | 138.9 | 138.9 | 138.9 | (138.8) | (138.8) | 137.8 |
| 12b | 77.4 | 77.4 | 77.5 | 77.5 | ( 76.1) | ( 76.0) | 76.2 |
| 13 | 25.5 | 25.5 | 25.5 | 25.5 | 30.3 | 30.3 | 24.4 |
| 1' | 71.1 | ( 71.5) | 71.1 | ( 71.5) | 71.1 | ( 71.5) | |
| 2' | 38.9 | 36.8 | 38.8 | 36.8 | 38.9 | 36.7 | |
| 3' | 71.3 | ( 74.6) | 71.2 | ( 74.6) | 71.3 | ( 74.6) | |
| 4' | 89.3 | ( 76.8) | 88.7 | ( 76.8) | 89.4 | ( 76.8) | |
| 5' | 74.4 | ( 74.5) | 74.5 | ( 74.5) | 74.4 | ( 74.5) | |
| 6' | 18.4 | 17.4 | 18.3 | 17.4 | 18.4 | 17.5 | |
| 1" | 95.2 | 91.4 | 99.6 | 91.4 | 95.2 | 91.4 | |
| 2" | 142.1 | ( 71.2) | 28.2 | ( 71.2) | 142.1 | ( 71.2) | |
| 3" | 127.3 | 40.0 | 33.4 | 40.0 | 127.3 | 40.0 | |
| 4" | 195.1 | 207.6 | 209.2 | 207.6 | 195.1 | 207.6 | |
| 5" | 71.6 | ( 77.7) | 72.0 | ( 77.7) | 71.6 | ( 77.7) | |
| 6" | 15.2 | 16.2 | 14.8 | 16.2 | 15.2 | 16.2 | |

| Carbon Assignment | M II 190 Y 1 | M II 190 Y 2 | M II 190 Y 3 | M II 190 Y 4 |
|---|---|---|---|---|
| 1''' | 92.4 | 92.5 | 92.6 | 92.6 |
| 2''' | 24.7 | 24.8 | 24.8 | 24.8 |
| 3''' | 24.5 | 24.6 | 24.7 | 24.7 |
| 4''' | 76.2 | 76.2 | 74.7 | 74.7 |
| 5''' | 67.0 | 67.0 | 67.2 | 67.2 |
| 6''' | 17.1 | 17.1 | 17.1 | 17.1 |
| 1'''' | 95.3 | 95.3 | 99.0 | 99.0 |
| 2'''' | 143.0 | 143.0 | 28.4 | 28.4 |
| 3'''' | 127.3 | 127.3 | 33.5 | 33.5 |
| 4'''' | 196.7 | 196.7 | 210.8 | 210.8 |
| 5'''' | 70.7 | 70.7 | 71.2 | 71.2 |
| 6'''' | 15.1 | 15.2 | 14.9 | 14.9 |

(Notes)

* Measured in d-$CHCl_3$ with tetramethyl silane (TMS) used as internal standard. The figures in the table are in ppm. The assignments are tentative and the parenthesized figures may be replaced in the future.

** Imamura, N. et al: J. Antibiotics, 35, 602-608 (1982)

Table 4 Assignment of Chemical Shifts in $^1$H-NMR spectra (400 MHz, in d-$CHCl_3$)

| Proton Assignment | MH 190 Y1 | MH 190 Y2 | MH 190 Y3 | MH 190 Y4 | MH 190 Y1-1 | MH 190 Y2-1 | MH 190 Y3-1 |
|---|---|---|---|---|---|---|---|
| 2-Hax | 2.51 d | 2.51 d | 2.51 d | 2.51 d | 2.63 d | 2.63 d | 2.63 d |
| 2-Heq | 3.18 dd | 3.19 dd | 3.20 dd | 3.20 dd | 2.95 dd | 2.95 dd | 2.95 dd |
| 3-$CH_3$ | 1.41 s | 1.41 s | 1.41 s | 1.41 s | 1.30 s | 1.30 s | 1.30 s |
| 4-Hax | 1.85 d | 1.84 d | 1.84 d | 1.84 d | 1.84 dd | 1.84 dd | 1.84 dd |
| 4-Heq | 2.29 dd | 2.29 dd | 2.29 dd | 2.29 dd | 2.27 dd | 2.28 dd | 2.27 dd |
| 5-H | 6.45 d | 6.45 d | 6.45 d | 6.45 d | 6.40 d | 6.40 d | 6.40 d |
| 6-H | 6.91 d | 6.91 d | 6.91 d | 6.91 d | 6.90 d | 6.90 d | 6.90 d |
| 8-OH | 12.29 s | 12.30 s | 12.30 s | 12.30 s | 12.28 s | 12.28 s | 12.28 s |
| 10-H | 7.88 d | 7.89 d | 7.88 d | 7.89 d | 7.89 d | 7.90 d | 7.89 d |
| 11-H | 7.62 d | 7.61 d | 7.62 d | 7.61 d | 7.62 d | 7.62 d | 7.62 d |
| OH | 4.33 s<br>4.59 s | 4.32 s<br>4.59 s | 4.35 s<br>4.59 s | 4.35 s<br>4.59 s | 3.58 d<br>3.91 s<br>4.99 s | 3.52 d<br>3.88 s<br>4.99 s | 3.52 d<br>3.89 s<br>4.99 s |
| 1'-H | 4.89 dd | 4.97 dd | 4.89 dd | 4.97 dd | 4.89 dd | 4.97 dd | 4.89 dd |
| 2'-Hax | 1.38 ~ 1.48 m | 1.43 ddd | 1.35 ~ 1.50 m | 1.35 ~ 1.45 m | 1.38 ~ 1.48 m | 1.44 ddd | ~ 1.4 m |
| 2'-Heq | 2.57 ddd | 2.45 ddd | 2.55 ddd | 2.40 ~ 2.55 m | 2.57 ddd | 2.43 ddd | 2.55 ddd |
| 3'-H | 3.91 dddd | 3.81 ddd | 3.86 dddd | 3.81 ddd | 3.91 dddd | 3.81 ddd | 3.86 dddd |
| 3'-OH | 4.26 d | — | 4.52 d | — | 4.26 d | — | 4.52 d |
| 4'-H | 3.21 dd | 3.48 t | 3.15 dd | 3.48 t | 3.21 dd | 3.49 t | 3.15 dd |
| 5'-H | 3.58 dq | 3.57 dq | 3.56 dq | 3.57 dq | 3.58 dq | 3.57 dq | 3.56 dq |
| 5'-$CH_3$ | 1.39 d | 1.40 d | 1.38 d | 1.40 d | 1.40 d | 1.40 d | 1.38 d |
| 1''-H | 5.38 d | 5.19 d | 5.18 t | 5.19 d | 5.38 d | 5.19 d | 5.18 t |
| 2''-H | 6.85 dd | — | — | — | 6.85 dd | — | — |
| 2''-Hax | — | — | ~ 2.1 m | — | — | — | 2.08 dddd |
| 2''-Heq | — | 4.34 ddd | 2.35 ~ 2.55 m | 4.34 ddd | — | 4.34 ddd | ~ 2.43 m |
| 3''-H | 6.15 d | — | — | — | 6.15 d | — | — |
| 3''-$H_A$ | — | 2.68 m | 2.35 ~ 2.55 m | 2.67 m | — | 2.67 m | 2.45 ~ 2.55 m |
| 3''-$H_B$ | — | 2.64 m | 2.35 ~ 2.55 m | 2.64 m | — | 2.63 m | 2.45 ~ 2.55 m |
| 5''-H | 4.76 q | 4.72 q | 4.50 q | 4.73 q | 4.76 q | 4.72 q | 4.50 q |
| 5''-$CH_3$ | 1.45 d | 1.38 d | 1.36 d | 1.39 d | 1.45 d | 1.38 d | 1.36 d |

Table 4 (continued)

| Proton Assignment | MH190 Y1 | MH190 Y2 | MH190 Y3 | MH190 Y4 | MH190 Y1-1 | MH190 Y2-1 | MH190 Y3-1 |
|---|---|---|---|---|---|---|---|
| 1''-H | 5.26 dd | 5.26 dd | 5.27 dd | 5.26 dd | | | |
| 2''-$H_A$ | 2.04 m | 2.03 m | 2.03 m | 2.03 m | | | |
| 2''-$H_B$ | 1.49 m | 1.49 m | ~1.46 m | ~1.46 m | | | |
| 3''-$H_A$ | 1.92 m | 1.92 m | ~1.87 m | ~1.87 m | | | |
| 3''-$H_B$ | 1.90 m | 1.91 m | ~1.87 m | ~1.87 m | | | |
| 4''-H | 3.70 ddd | 3.70 ddd | 3.66 m | 3.66 m | | | |
| 5''-H | 4.25 dq | 4.25 dq | 4.22 dq | 4.22 dq | | | |
| 5''-$CH_3$ | 1.29 d | 1.29 d | 1.27 d | 1.27 d | | | |
| 1'''-H | 5.27 d | 5.27 d | 5.09 t | 5.09 t | | | |
| 2'''-H | 6.89 dd | 6.89 dd | — | — | | | |
| 2'''-$H_{ax}$ | — | — | 2.10 dddd | 2.10 dddd | | | |
| 2'''-$H_{eq}$ | — | — | 2.36 dddd | 2.36 dddd | | | |
| 3'''-H | 6.09 d | 6.09 d | — | — | | | |
| 3'''-$H_A$ | — | — | 2.35~2.55 m | 2.35~2.55 m | | | |
| 3'''-$H_B$ | — | — | 2.35~2.55 m | 2.35~2.55 m | | | |
| 5'''-H | 4.55 q | 4.55 q | 4.29 q | 4.29 q | | | |
| 5'''-$CH_3$ | 1.37 d | 1.37 d | 1.27 d | 1.27 d | | | |

(Note)

* The figures in the table represent respective signals in ppm with tetramethyl silane (TMS) used as internal standard. The multiplicity of respective signals is indicated by s (singlet), d (doublet), t (triplet), q (quartet) and m (multiplet)

Several of the physicochemical properties of the compounds within the scope of substance MH190 in accordance with the present invention are shown in Table 5.

Table 5 Physicochemical Properties of Substance MH190

| | MH190 Y1 | MH190 Y2 | MH190 Y3 | MH190 Y4 | MH190 Y1-1 | MH190 Y2-1 | MH190 Y3-1 |
|---|---|---|---|---|---|---|---|
| 1. Appearance | orange yellow powder | orange yellow powder | orange yellow powder | orange yellow powder | orange yellow powder | orange yellow powder | orange yellow powder |
| 2. Elemental analysis (1) Found | C : 63.05<br>H : 5.96<br>O : 30.99 | C : 62.80<br>H : 5.95<br>O : 31.25 | C : 62.12<br>H : 6.64<br>O : 31.24 | C : 62.65<br>H : 6.20<br>O : 31.15 | C : 62.29<br>H : 5.52<br>O : 32.19 | C : 62.11<br>H : 5.55<br>O : 32.34 | C : 62.40<br>H : 5.59<br>O : 32.01 |
| (2) Calculated | C : 62.92<br>H : 5.89<br>O : 31.19<br>(for $C_{43}H_{48}O_{16}$) | C : 62.92<br>H : 5.89<br>O : 31.19<br>(for $C_{43}H_{48}O_{16}$) | C : 62.61<br>H : 6.35<br>O : 31.03<br>(for $C_{43}H_{52}O_{16}$) | C : 62.77<br>H : 6.12<br>O : 31.11<br>(for $C_{43}H_{50}O_{16}$) | C : 62.41<br>H : 5.41<br>O : 32.18<br>(for $C_{31}H_{32}O_{12}$) | C : 62.41<br>H : 5.41<br>O : 32.18<br>(for $C_{31}H_{32}O_{12}$) | C : 62.20<br>H : 5.73<br>O : 32.07<br>(for $C_{31}H_{34}O_{12}$) |
| 3. Molecular weight (1) FD-MS, m/z | — | — | — | — | 596(M)$^+$ | 596(M)$^+$ | 598(M)$^+$ |
| (2) Calculated | 820.84 | 820.84 | 824.87 | 822.86 | 596.59 | 596.59 | 598.60 |
| 4. Melting point (°C) | 149~152 (with decomp.) | 164~166 (with decomp.) | 142~145 (with decomp.) | 152~155 (with decomp.) | 166~168 (with decomp.) | 180~182 (with decomp.) | 158~160 (with decomp.) |
| 5. Specific rotation $[\alpha]_D^{24}$ | +77° (c 0.4, in $CHCl_3$) | +96° (c 0.2, in $CHCl_3$) | −53.3° (c 0.3, in $CHCl_3$) | +10.0° (c 0.2, in $CHCl_3$) | +117° (c 0.2, in $CHCl_3$) | +148° (c 0.2, in $CHCl_3$) | +43° (c 0.2, in $CHCl_3$) |
| 6. Absorption spectrum for UV and visible bands λmax nm($E_{1cm}^{1\%}$) (1) in 0.1N HCl-90% MeOH | 218 (372)<br>317 (59)<br>425 (64) | 218 (358)<br>317 (65)<br>425 (68) | 218 (318)<br>316 (58)<br>425 (65) | 218 (299)<br>318 (55)<br>425 (63) | 218 (488)<br>316 (84)<br>425 (89) | 218 (447)<br>316 (85)<br>425 (92) | 220 (485)<br>318 (110)<br>425 (95) |
| (2) in 0.1N NaOH-90% MeOH | 215 (1120)<br>269 (158)<br>520 (65) | 215 (1165)<br>~280s (133)<br>520 (68) | 215 (1198)<br>~270s (260)<br>520 (67) | 215 (1055)<br>270 (144)<br>520 (70) | 213 (1870)<br>275 (174)<br>520 (92) | 216 (1830)<br>270 (197)<br>520 (96) | 216 (1890)<br>~270 (187)<br>520 (99) |

Table 5 (continued)

| | MH190 Y1 | MH190 Y2 | MH190 Y3 | MH190 Y4 | MH190 Y1-1 | MH190 Y2-1 | MH190 Y3-1 |
|---|---|---|---|---|---|---|---|
| 7. IR spectrum | Fig. 2 | Fig. 3 | Fig. 4 | Fig. 5 | Fig. 6 | Fig. 7 | Fig. 8 |
| 8. $^1$H-NMR spectrum | Table 4 | Table 4 | Table 4 | Table 4 | Table 4 | Table 4 | Table 4 |
| 9. $^{13}$C-NMR spectrum | Table 3 | Table 3 | Table 3 | Table 3 | Table 3 | Table 3 | Table 3 |
| 10. TLC Rf value* | 0.51 | 0.64 | 0.44 | 0.59 | 0.31 | 0.40 | 0.30 |
| 11. Solubility | | | | | | | |
| (1) Soluble | $CHCl_3$, ethyl acetate, butyl acetate, methanol, acetonitrile, acetone, dimethyl sulfoxide | $CHCl_3$, ethyl acetate, butyl acetate, methanol, acetonitrile, acetone, dimethyl sulfoxide | $CHCl_3$, ethyl acetate, butyl acetate, methanol, acetonitrile, acetone, dimethyl sulfoxide | $CHCl_3$, ethyl acetate, butyl acetate, methanol, acetonitrile, acetone, dimethyl sulfoxide | $CHCl_3$, ethyl acetate, butyl acetate, methanol, acetonitrile, acetone, dimethyl sulfoxide | $CHCl_3$, ethyl acetate, butyl acetate, methanol, acetonitrile, acetone, dimethyl sulfoxide | $CHCl_3$, ethyl acetate, butyl acetate, methanol, acetonitrile acetone, dimethyl sulfoxide |
| (2) Sparingly soluble | $H_2O$, hexane, petroleum ether | $H_2O$, hexane, petroleum ether | $H_2O$, hexane, petroleum ether | $H_2O$, hexane, petroleum ether | $H_2O$, hexane, petroleum ether | $H_2O$, hexane, petroleum ethet | $H_2O$, hexane, petroleum ether |
| 12. Stability | instable against light, acid and alkali<br>changed to MH190 Y2 on silica gel | instable against light, acid and alkali | instable against light, acid and alkali | instable against light, acid and alkali | instable against light, acid and alkali<br>changed to MH190 Y2 on silica gel | instable against light, acid and alkali | instable against light, acid and alkali |

(Note)

HPTLC plate was Kieselgel® 60 $F_{254}$ (Merck, Art 5628).

Developing solvent was a 10:10:0.2 mixture of chloroform, ethyl acetate and acetic acid.

The benz[a]anthraquinone compounds of the present invention have so far been obtained solely by cultivation of microorganisms. They may however be produced by chemical synthetic, microbial or enzymatic modification of analogous compounds, or by fully chemical synthetic procedures.

In producing the benz[a]anthraquinone compounds of the present invention by cultivation of a microorganism, one that belongs to the genus Streptomyces and which has the ability of producing benz[a]-anthraquinone compounds is used. It has been established that the strain MH190-16F3 has the ability to

produce substance MH190, but it should be noted that other suitable strains may be isolated from nature by conventional techniques for isolating antibiotic-producing microorganisms.

It is also possible to enhance the substance MH190 producing ability of the strain MH190-16F3 and other MH190 producing microorganisms by treating them with radiation or nitrosoguanidine, or by cell fusion or DNA recombinant technology, or by subjecting them to other suitable treatments.

The strain MH190-16F3 that has been identified as a microorganism of the genus Streptomyces having the ability to produce the benz[a]anthraquinone compound or substance MH190 is specifically described below.

(1) Origin and Accession Number

The strain MH190-16F3 is an actinomycete isolated by the Institute of Microbial Chemistry in March 1984 from soil sampled in Tobata-ku, Kita-Kyushu-shi, Japan. This strain was deposited with the Fermentation Research Institute, the Agency of Industrial Science and Technology on December 7, 1985 and was given the accession number, FERM-P No. 7985.

(2) Mycological properties

A. Morphology

Strain MH190-16F3 as observed under a microscope has short hooked or spiral aerial mycelia extending from branched aerial hyphae, but no whirl is observed. Mature spore chains have a detectable chain of 10-20 spores. The spore is in the size range of about 0.6 - 0.7 x 1.0 - 1.1 µm. The spores are smooth-surfaced.

B. State of growth on media

In the following description, color standards are bracketed and are in accordance with the Color Harmony Manual of Container Corporation of America.

(a) Sucrose-nitrate agar medium (incubated at 27° C)

Adhering light olive gray to light brown gray [2ge, Covert Tan - 2ig, Slate Tan] aerial mycelia form on a colorless to pale yellow growth. No soluble dye observed.

(b) Glucose-asparagine agar medium (incubated at 27° C)

Adhering brown white to light brown gray [1fe, Griege - 2fe, Covert Gray] aerial mycelia form on a dull yellow brown [2ne, Mustard Gold - 3ne, Topaz] growth. Light reddish yellow soluble dye forms.

(c) Glycerin-asparagine agar medium (incubated in ISP medium 5 at 27° C)

Adhering light olive gray [1½ ge, Lt Olive Gray] to light brown gray [2ge, Covert Tan - 2it, Slate Tan] aerial mycelia form on a pale yellow brown [3ni, Clove Brown] to light brown [4pi, Oak Brown] growth. Light gray soluble dye forms.

(d) Starch-inorganic salt agar medium (incubated in ISP medium 4 at 27° C)

Adhering light gray [2fe, Covert Gray] to light olive gray [1½ ge, Lt. Olive Gray] aerial mycelia form on a pale yellow to dark yellow brown [2pl, Mustard Brown] growth. Soluble dye with a brown tinge forms.

(e) Tyrosine agar medium (incubated in SIP medium 7 at 27° C)

Adhering light olive gray [1½ ge, Lt Olive Gray] to light brown gray [2ge, Covert Tan - 2ig, Slate Tan] aerial mycelia form on a yellow brown to light brown [4pg, Dk Luggage Tan - 4pi, Oak Brown] growth. Light brown soluble dye forms.

(f) Nutrient agar medium (incubated at 27° C)

Pale yellow [2go, Bamboo] growth with no adhering aerial mycelia. No soluble dye observed.

(g) Yeast-malt agar medium (incubated in ISP medium 2 at 27° C)

Adhering light brown gray [2ig, Slate Tan] to olive gray [1½ ig, Olive Gray - lih, Olive Gray] aerial mycelia form on a pale yellow brown [3ie, Camel - 31e, Cinnamon] growth. Soluble dye with a brown tinge observed.

(h) Oatmeal agar medium (incubated in ISP medium 3 at 27° C)

Adhering light olive gray to olive gray [1½ ig, Olive Gray] aerial mycelia form on a pale yellow to pale yellow brown [3ie, Camel] growth. Soluble dye with a brown tinge observed.

(i) Glycerin-nitrate agar medium (incubated at 27° C)

Adhering white to brown white [3cb, Sand] aerial mycelia form on a pale yellow brown [3ne, Topaz] to yellow brown [3pg, Golden Brown] growth. Yelow brown soluble dye produced.

(j) Starch agar medium (incubated at 27° C)

Thin film of white aerial mycelia forms on a dull yellow [2ne, Mustard Gold - 3ne, Topaz] to yellow brown [4pi, Oak Brown] growth. Dull yellow to dark yellow soluble dye observed.

(k) Calcium malate agar medium (incubated at 27° C)

Adhering brown white to light gray aerial mycelia form on a colorless growth. Soluble dye with

a brown tinge forms.

(ℓ) Cellulose (incubated at 27° C)

No growth observed in the 3 weeks subsequent to incubation.

(m) Gelatin stab culture

In incubation on a simple gelatin medium at 20° C, a small amount of white aerial mycelia form on a pale yellow to pale yellow brown growth, with the formation of a slightly brownish soluble dye. In incubation on a glucose-peptone gelatin medium at 27° C, no adhering aerial mycelia observed on a pale yellow growth. No soluble dye observed.

(n) Skimmed cow's milk (incubated at 37° C)

No adhering aerial mycelia observed on a pale yellow growth. No soluble dye observed.

(3) Physiological properties

a. Growth temperature range

Growth test was conducted on glucose-asparagine agar (1.0% glucose, 0.05% L-asparagine, 0.05% dipotassium phosphate, 3.0% agar, pH 7.0) at varying temperatures of 20° C, 24° C, 27° C, 30° C, 37° C and 50° C. Except at 50° C, the microorganism grew at each of the temperatures tested, but an optimal temperature would be in the range of 27 - 30° C.

b. Liquefaction of gelatin (on (i) 15% simple gelatin at 20° C, and (ii) on glucose-peptone gelatin at 27° C)

With the simple gelatin medium, liquefaction started at about 13 days of incubation, and the action of liquefaction was moderate.

With the glucose-peptone gelatin medium, slight liquefaction started at about 13 days of incubation and the action of liquefaction was very weak.

c. Hydrolysis of starch (incubated either on starch-inorganic salt agar medium or on starch agar medium at 27° C)

With both mediums, hydrolysis of starch was observed starting on the second day of incubation, and the action of hydrolysis was moderate to strong.

d. Coagulation and peptonization of skimmed cow's milk (incubated on skimmed cow's milk at 37° C)

Coagulation of skimmed cow's milk started at about 4 days of incubation and was soon completed, followed by peptonization. The action of peptonization was moderate to strong.

e. Formation of malanine-like pigment (with (i) tryptone-yeast broth, ISP medium 1, (ii) peptone-yeast-iron agar, ISP medium 6, and (iii) tyrosine agar, ISP medium 7, incubated at 27° C)

No melanine-like pigment observed in either medium.

f. Utilization of carbon sources (on Pridham-Gottlieb agar medium, ISP medium 9, incubated at 27° C)

The microorganism grew utilizing L-arabinose, glucose, D-fructose, inositol, rhamnose and D-mannitol, but not utilizing sucrose. The microorganism would probably utilize D-xylose and raffinose.

g. Dissolution of calcium malate (on calcium malate agar at 27° C)

No dissolution of calcium malate was observed.

h. Reduction of nitrate (in aqueous solution of peptone containing 0.1% $KNO_3$, ISP medium 8, at 27° C)

Both positive and negative cases occurred in a cyclic test.

The above observations can be summarized as follows:

morphologically, the strain MH190-16F3 has no sporangium or whirl and has spiral aerial mycelia. The microorganism has smooth-surfaced spores. It grows on a variety of media, providing light brown gray to olive gray aerial mycelia on pale yellow to light brown growths. Yellow brown to light brown growths occur on many media. The formation of a melanine-like pigment is negative in all of the media used. Moderate to strong hydrolysis of starch occurs. The ability of the microorganism to decompose protein is such that it liquefies gelatin moderately to weakly, and coagulates and peptonizes skimmed cow's milk moderately to strongly.

The cells of this microorganism contain an LL-form of 2,6-diaminopimelic acid. This fact, taken together with the above described properties, clearly shows that the strain MH190-16F3 belongs to the genus Streptomyces.

Utilizing these mycological properties as a guide, Streptomyces nodosus ((1) International Journal of Systematic Bacteriology, 18, 353, 1968, (2) S.A. Waksman; The Actinomycetes, Vol. 2, 250, 1961, and (3) Journal of Bacteriology, 85, 436, 1963) is considered to be most homologous to strain MH190-16F3.

Substances analogous to the substance produced by the strain MH190-16F3 are produced by the following two microorganisms: Vineomycin-producing streptomyces matensis subp. vineus (The Journal of Antibiotics, 30, 908, 1977) and Ayamacin-producing streptomyces flaveolus-like 0-80 strain (The Journal of

Antibiotics, 13, 391, 1960). Streptomyces matensis subsp. vineus is distinguished from the strain MH190-16F3 in that the former produces spores with prickly surfaces. The strain 0-80 is distinguished from MH190-16F3 in that the former produces a melanine-like pigment and grows utilizing sucrose. The strain MH190-16F3 is also distinguished from substance TA-435 producing Streptomyces s.p. 0-80-like strain (The Journal of Antibiotics Ser. B. 17, 245, 1964) in that the latter produces a melanine-like pigment, dissolves calcium malate, and does not utilize inositol, mannitol or fructose.

In order to confirm these assumptions, the present inventors obtained the ISP strain of the seemingly most homologous Streptomyces nodosus and compared it with MH190-16F3. The results of comparison are summarized in the following table together with the description in the literature.

Table 6

| | MH190-16F3 | Streptomyces nodosus | |
|---|---|---|---|
| | | IMC S-0125(ISP5109) | Description in the literature |
| Whirl formation | − | − | − |
| Spiral formation | + | + | + |
| Spore surface | smooth | smooth | smooth |
| Color of aerial mycelium | light brown gray ∿ olive gray | light brown gray ∿ olive gray | predominantly grav ** (gray white ∿ olive gray) |
| Color of growth | pale yellow ∿ light brown | pale yellow ∿ light brown | gray yellow ∿ dark olive brown |
| Soluble dye | yellow brown ∿ light brown | yellow brown ∿ light brown | yellow orange ∿ red |
| Formation of melanine-like pigment | | | |
| ISP medium 1 | − | − | − |
| ISP medium 6 | − | − | − |
| ISP medium 7 | − | − | − |
| Hydrolysis of starch | + | + | + ** |
| Coagulation of cow's milk | + | + | |
| Peptonization of cow's milk | + | + | + ** |
| Liquefaction of gelatin | | | |
| simple gelatin | + | + | + ** (medium unknown) |
| glucose-peptone gelatin | ∓ (very weak) | − | |

Table 6 (continued)

| | MH190-16F3 | Streptomyces nodosus | |
|---|---|---|---|
| | | IMC S-0125 (ISP5109) | Description in the literature |
| Reduction of nitrate | ± (positive or negative in cyclic test) | + | + |
| Utilization of carbon source * | | | |
| Glucose | + | + | + |
| L-arabinose | + | d | d |
| D-xylose | (+) | (+) | + |
| D-fructose | + | + | + |
| Sucrose | − | − | − |
| Inositol | + | (+) | + |
| Rhamnose | + | (+) | + |
| Raffinose | (+) | d | − |
| D-mannitol | + | + | + |

* +: utilized, (+): probably utilized, d: unclear, −: not utilized

Literature: Description with two asterisks is found in S.A. Waksman, The Actinomyces, Vol. II, p. 250, 1961, and description without any asterisk is found in International Journal of Systematic Bacteriology, 18, p. 353, 1968.

As the above table shows, the properties of strain MH190-16F3 are in complete agreement with those of Streptomyces nodosus except for slight differences in respect to the utilization of L-arabinose and raffinose. As mentioned in the section of "Morphology", MH190-16F3 forms aerial mycelia in a characteristic short hooked or spiral form (the spiral would more accurately be described as being like a knot in a piece of string). In this respect, MH190-16F3 is very close to the photographs of Streptomyces nodosus shown in

the literature. In addition, the two microorganisms looked identical to each other in electron micrographs.

With all the observations taken together, strain MH190-16F3 is believed to be most homologous to Streptomyces nodosus, and therefore, said strain has been identified as Streptomyces nodosus MH190-16F3.

The benz[a]anthraquinone compounds, or substances MH190, can be produced by aerobically cultivating a MH190-producing microorganism of the genus Streptomyces on a suitable medium and recovering the end product from the culture.

The medium may contain any of the nutrient sources that can be utilized by the MH190-producing microorganism. Usable carbon sources include glycerin, glucose, sucrose, maltose, dextrin, starch and fats and oils. Usable nitrogen sources include organics such as soybean meal, cottonseed oil cake, meat extract, peptone, dried yeast, yeast extract and corn steep liquor, and inorganics such as ammonium salts and nitrates, for example, ammonium sulfate, sodium nitrate and ammonium chloride. If necessary, inorganic salts such as sodium chloride, potassium chloride, phosphates and heavy metal salts may be added to the medium. In order to prevent foaming during fermentation, suitable anti-foaming agents such as silicone and soybean oil may be added in suitable amounts as effected in the usual practice.

Aerobic liquid submerged culture in the most appropriate method as is commonly employed in the production of antibiotics. A suitable temperature for cultivation ranges from 20 to 35 °C, with the 25 - 30 °C range being preferred. The amount of substance MH190 produced by this method, whether it is a shake culture or culture under aeration and agitation, reaches a peak in 2 - 4 days.

The above procedures yield a culture in which the substance MH190 has accumulated. Part of the substance MH190 is present in the cells of the cultured microorganism but most of the substance is present in the supernatant of the culture.

Any suitable method may be used to recover the substance MH190 from the culture. One method depends on the principles of extraction. More specifically, substance MH190 in the culture supernatant may be extracted with water-immiscible solvents for MH190 (see above), such as ethyl acetate, butyl acetate, and chloroform. If the substance MH190 is present in the cells of the cultured microorganism, the cells are collected by filtration, centrifugation or other suitable techniques and the substance MH190 may be recovered by treatment with ethyl acetate, chloroform, methanol, ethanol, butanol, acetone or methyl ethyl ketone. The culture may be immediately subjected to the above procedures of extraction without isolating the cells. The cells may be disrupted prior to extraction. The counter-current distribution method may be included within the category of extraction techniques.

Another method that can be used to recover the substance MH190 from the culture depends on the principles of adsorption, wherein an already liquid material containing the substance MH190, such as a culture filtrate or an extract that has been obtained by the first method, is subjected to column chromatography or liquid chromatography using a suitable adsorbent, say, activated carbon, alumina, silica gel or Dia-Ion HP20 (Mitsubishi Chemical Industries Limited), and the adsorbed substance MH190 is eluted with a suitable solvent. The eluate is subsequently concentrated to dryness under vacuum to obtain a crude product of the substance MH190.

The crude product of substance MH190 may be purified by performing the required number of cycles of the extraction and adsorption processes described above, which may be combined as required, optionally followed by recrystallization. Suitable purification techniques that may be used in combination include column chromatographic processes using adsorbents and gel permeation media such as silica gel, Sephadex LH 20 and Dia-Ion HP 20 (Mitsubishi Chemical Industries Limited), liquid chromatography using suitable solvents, the counter-current distribution method and thin-layer chromatography. A specific purification process may proceed as follows: the crude powder of substance MH190 is dissolved in a small amount of chloroform; the solution is loaded onto a silica gel column and fractions are eluted with a suitable solvent; those fractions containing the desired substance are combined and concentrated under vacuum; the concentrate is subjected to thin-layer chromatography and the desired components are recovered to obtain a substantially homogenous product. This product may be further purified by high-performance liquid chromatography or crystallization from a suitable solvent.

The benz[a]anthraquinone compounds or substance MH190, of the present invention have cancer-control activities and are therefore useful as medicaments. Some of the biological activities of this substance are described below.

1) Action against multi-drug resistant cells (P388/ADR)

The substance MH190 of the present invention are capable of inhibiting the growth of cultured Adriamycin-sensitive P388 leukemia cells (P388/S) and Adriamycin-resistant P388 leukemia cells (P388/ADR) in very low concentrations. The median inhibition concentration ion ($IC_{50}$) of the substance is substantially the same for both P388/ADR and P388/S, suggesting the effectiveness of the substance

against the resistant cells.

Table 7

| Compound | IC50 (μg/mℓ) P388/S | IC50 (μg/mℓ) P388/ADR | Ratio (P388/ADR to P388/S) |
|---|---|---|---|
| Adriamycin | 0.012 | 0.38 | 31.7 |
| MH190Y1 | 0.067 | 0.056 | 0.84 |
| MH190Y2 | 0.080 | 0.067 | 0.84 |
| MH190Y3 | 0.072 | 0.060 | 0.83 |
| MH190Y4 | 0.070 | 0.060 | 0.86 |
| MH190Y1-1 | 0.175 | 0.09 | 0.51 |
| MH190Y2-1 | 0.130 | 0.074 | 0.57 |

2) Anti-tumor activity

L1210 leukemia cells (1 x $10^5$ per animal) were transplanted intraperitoneally into $CDF_1$ mice. Immediately after the transplantation, a solution of MH190 Y2 was injected intraperitoneally for 10 days in a dose of 0.25 m. The percentage life prolongation of each of the treated mice was checked and the results are shown below, with the number of days of survival of control mice to which physiological saline had been administered being taken as 100.

Table 8

| Dose mg/Kg/dat | Percentage life prolongation |
|---|---|
| 5 | 175 |
| 2.5 | 151 |
| 1.25 | 127 |
| 0.625 | 120 |
| 0.313 | 127 |
| 0.156 | 102 |

In the MH190 Y2 treated group, appreciable anti-tumor activities were exhibited by administration of doses ranging from 0.313 to 5 mg/kg/day.

3) Acute toxicity ($LD_{50}$)

The $LD_{50}$ of MH190 Y2 for a single intraperitoneal injection into mice was 6.25 - 12.5 mm/kg.

Examples

Example 1

(1) Preparation of seed culture

A medium having the following composition was used.

| | |
|---|---|
| Galactose | 2.0% |
| Dextrin | 2.0% |
| Bactosoyton (Difco) | 1.0% |
| Corn steep liquor | 0.5% |
| Ammonium sulfate | 0.2% |
| Calcium carbonate | 0.2% |

(pH 7.4 before sterilization)

A portion (110 mℓ) of this medium was poured into a 500-mℓ Erlenmeyer flask and sterilized. The medium was then inoculated with a loopful of a slant culture of strain MH190-16F3. The medium was shake-cultured in a rotary shaker at 27° C for 3 days to prepare a seed culture.

(2) Fermentation

A medium having the same composition as that used in the preparation of the seed culture was used in fermentation.

A 30-ℓ jar fermenter was charged with 15 liters of the above medium, which was inoculated with 3

lots (330 mℓ) of the seed culture. Cultivation was conducted at 27° C for 48 hours under agitation at 200 rpm and aeration at a rate of 15 ℓ/min.

(3) Recovery of substance MH190 from the culture filtrate

The culture obtained in (2) was centrifuged and 8 liters of the supernatant was subjected to extraction with 8 liters of butyl acetate at pH 7.0. The upper layer was dehydrated with anhydrous sodium sulfate and evaporated to dryness under vacuum to obtain 770 mg of a black brown powder. The powder was dissolved in a small amount of chloroform and the solution was passed through a column packed with 30 g of silica gel (Kieselgel® 60 of Merck, 70-230 mesh). The column was eluted by density gradient of a mixture of chloroform and ethyl acetate. The fractions containing substance MH190 were collected and evaporated to dryness, thereby producing 125 mg of a crude powder of substance MH190. The predominant components of the fractions were MH190 Y1 and Y2.

(4) Recovery of substance MH190 from cultured cells

Methanol (6 liters) was added to about 2 liters of the cells in the culture, and after thorough agitation, the mixture was filtered to obtain a methanol extract. The extract was concentrated, and washed with a small amount of petroleum ether twice. Thereafter, the insoluble fractions were extracted with 1 liter of a mixture of water and butyl acetate (1:1). The upper layer was dehydrated with anhydrous sodium sulfate and evaporated to dryness to obtain 450 mg of a black brown powder. The powder was dissolved in a small amount of chloroform and the solution was passed through a column packed with 20 g of silica gel (Kieselgel® 60 of Merck, 70-230 mesh). The column was eluted as in (3) by density gradient of a mixture of chloroform and ethyl acetate. The so obtained fractions containing the substance MH190 were evaporated to dryness, thereby producing 25 mg of a crude powder of substance MH190. The predominant components of the powder were MH190 Y3 and Y4.

## Example 2

The crude powder (125 mg) obtained in Example 1(3) was dissolved in a small amount of chloroform and the solution was spread on 15 thin layers of silica gel (Kieselgel® 60 $F_{254}$ of Merck, 0.25 mm x 20 cm x 20 cm), which were eluted with a 100:2 mixture of chloroform and methanol. The separate MH190 Y1 and Y2 fractions were collected, eluted from the silica gel plates with a 20:1 mixture of chloroform and methanol, and evaporated to dryness. The resulting powders were dissolved in a 1:10 mixture of chloroform and methanol and passed through a column of "Sephadex LH 20" (1.5 x 30 cm) equilibrated with the same composition of chloroform-methanol mixture. The effluents were evaporated to dryness to obtain 32 mg of a yellow orange powder of MH190 Y1 and 45 mg of a yellow orange powder of MH190 Y2.

A portion of MH190 Y1 (15 mg) was dissolved in 5 mℓ of a mixture of 0.4% aqueous phosphoric acid and acetonitrile, and after leaving it to stand overnight at room temperature, the solution was subjected to extraction with chloroform. The chloroform layer was evaporated to dryness and a homogenous product of MH190 Y1-1 (18.2 mg) was obtained by subjecting the concentrate to reverse-phase HPLC under the following conditions:

Column: Nucleosil $5C_{18}$ of M. Nagel, 20$\phi$ x 300 mm;
Eluant: 0.4% aqueous phosphoric acid/acetonitrile (42:58);
Flow rate: 10 mℓ/min.

Under these conditions, MH190 Y1-1 was eluted out in 12 minutes.

A portion (15 mg) of MH190 Y2 was likewise dissolved in 5 mℓ of a mixture of 0.4% aqueous phosphoric acid and acetonitrile (40:60) and the solution was left to stand overnight at room temperature. After extraction with chloroform and evaporation to dryness, the solid was subjected to HPLC under the same conditions as used in the purification of MH190 Y1-1. By these procedures, 6.6 mg of MH190 Y2-1 was obtained. The product was eluted in 15 minutes.

## Example 3

The crude powder (25 mg) of the substance MH190 obtained in Example 1(4) was dissolved in a small amount of chloroform and the solution was spread on 4 thin layers of silica gel ("Kieselgel® 60 $F_{254}$" of Merck, 0.25 mm x 20 cm x 20 cm), which were developed with a mixture of chloroform, ethyl acetate and acetic acid (100:100:2) and the separating MH190 Y3 and Y4 fractions were collected individually. Each of these silica gels were eluted with a mixture of chloroform and methanol (20:1) and washed three times with an equal amount of water. The resulting yellow solutions were evaporated to dryness under vacuum to obtain 5.9 mg of a yellow orange powder of MH190 Y3 and 6.6 mg of a yellow orange powder of MH190 Y4.

A portion (5 mg) of MH190 Y3 was dissolved in a mixture of 0.4% aqueous phosphoric acid and acetonitrile (40:60) and the solution was left to stand overnight at room temperature. After extraction with chloroform and evaporation to dryness, the solid was isolated and purified by HPLC under the same conditions as shown in Example 2. A homogeneous product of MH190 Y3-1 was obtained in an amount of 2.2 mg. It was eluted in 12 minutes.

Brief Description of the Drawings

Fig. 1 is a flowsheet showing the sequence for determining the structure of substances MH190 of the present invention; and
Figs. 2 to 8 are sketches of the IR spectra (KBr tab.) of MH190 Y1, Y2, Y3, Y4, Y1-1, Y2-1 and Y3-1.

## Claims

**Claims for the following Contracting States: BE, CH, DE, FR, GB, IT, LI, LU, NL, SE**

1. A compound of the formula I

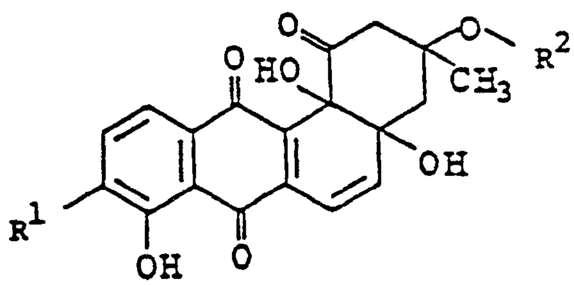

[I]

wherein $R^1$ represents one of the radicals having the following formulae:

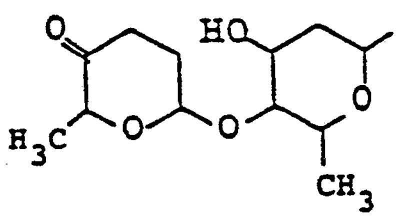

II

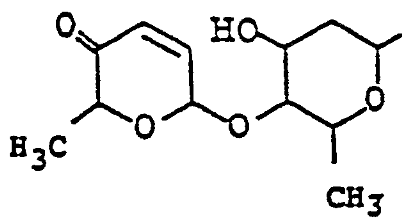

III

or

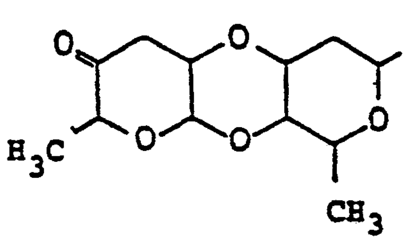

IV

and $R^2$ represents hydrogen or one of the radicals having the formulae

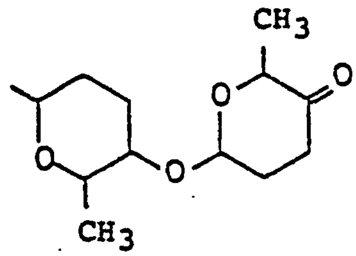

V

or

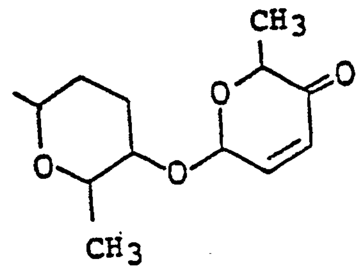

VI

2. The compound as defined in claim 1 wherein $R^1$ is the radical of the formula III and $R^2$ is the radical of the formula VI.

3. The compound as defined in claim 1 wherein $R^1$ is the radical of the formula IV and $R^2$ is the radical of the formula VI.

4. The compound as defined in claim 1 wherein $R^1$ is the radical of the formula II and $R^2$ is the radical of the formula V.

5. The compound as defined in claim 1 wherein $R^1$ is the radical of the formula IV and $R^2$ is the radical of the formula V.

6. The compound as defined in claim 1 wherein $R^1$ is the radical of the formula III and $R^2$ is hydrogen.

7. The compound as defined in claim 1 wherein $R^1$ is the radical of the formula IV and $R^2$ is hydrogen.

8. The compound as defined in claim 1 wherein $R^1$ is the radical of the formula II and $R^2$ is hydrogen.

9. A pharmaceutical composition which comprises as active ingredient a compound of the formula I as defined in claim 1 in association with a pharmaceutically acceptable carrier.

10. Use of a compound of the formula I as defined in claim 1 for the preparation of a medicament being effective against multi-drug resistant tumor cells.

11. The microorganism strain Streptomyces nodosus MH190-16F3 (FERM P-No. 7985).

12. A process for the preparation of a compound of the formula I

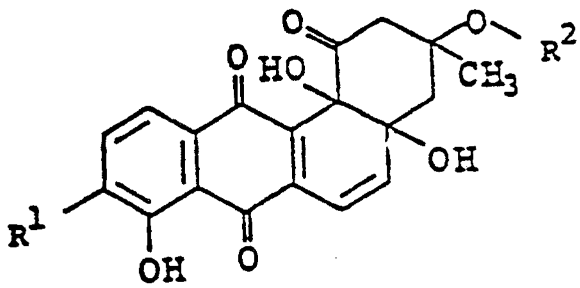

[I]

wherein $R^1$ represents one of the radicals having the following formulae:

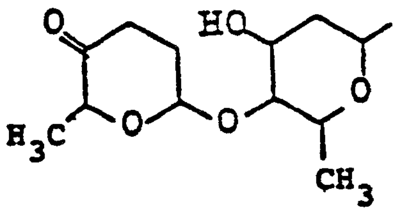

II

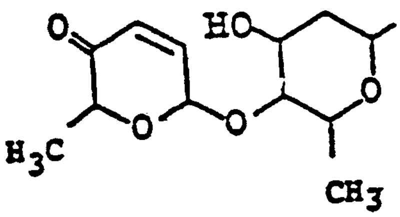

III

or

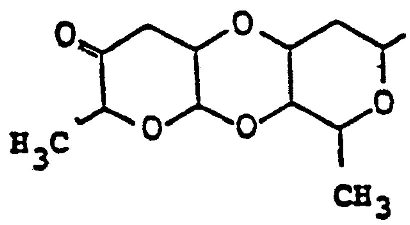

IV

and $R^2$ represents hydrogen or one of the radicals having the formulae

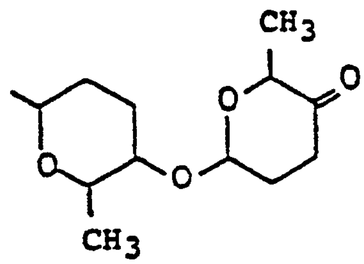

V

or

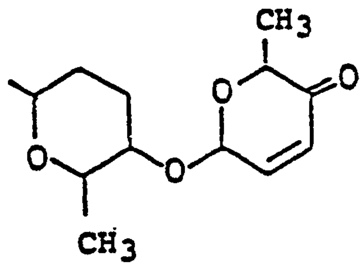

VI

which comprises aerobically cultivating a microorganism strain of the genus Streptomyes in a suitable culture medium and recovering the product from the culture broth and optionally from the mycel by conventional extraction and/or adsorption processes.

13. The process defined in claim 12 wherein the strain Streptomyces nodosus MH190-16F3 (FERM P No. 7985) is used.

14. The process as defined in claims 12 and 13 wherein the cultivation is carried through in a liquid submerged culture medium comprising nutritional carbon sources and nitrogen sources and optionally inorganic salts and/or antifoaming agents at a temperature of from 20 to 35°C and a cultivation time of from 2 to 4 days, the produced compounds are recovered from the culture broth by extraction with an organic waterimmiscible solvent and from the mycel with an aliphatic ester or ketone, a lower alkanol, or chloroform, and are recovered from the extracts, separated and purified by subjecting the extracts to column or liquid chromatography on a suitable adsorbent.

**Claims for the following Contracting State: AT**

1. A process for the preparation of a compound of the formula I

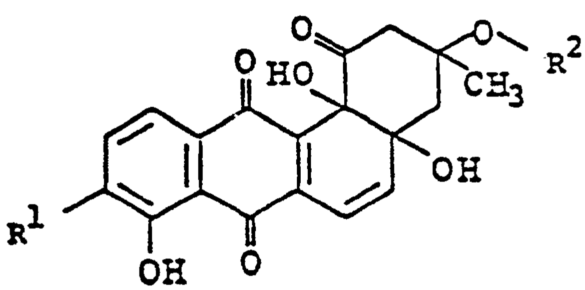

[I]

wherein $R^1$ represents one of the radicals having the following formulae:

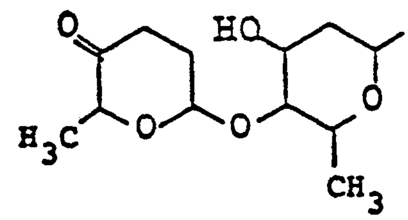

II

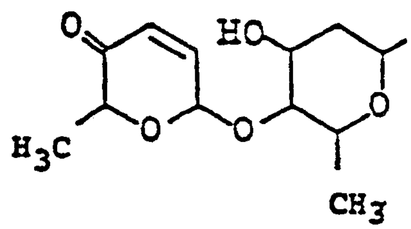

III

or

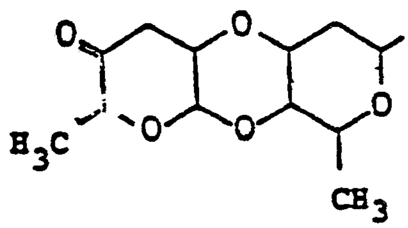

IV

and $R^2$ represents hydrogen or one of the radicals having the formulae

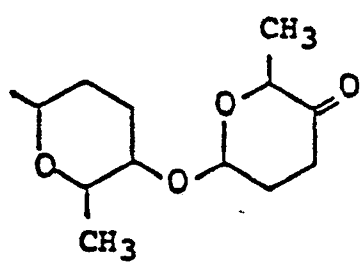

V

or

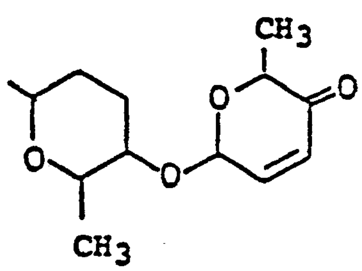

VI

which comprises aerobically cultivating a microorganism strain of the genus Streptomyes in a suitable culture medium and recovering the product from the culture broth and optionally from the mycel by conventional extraction and/or adsorption processes.

**2.** The process defined in claim 1 wherein the strain Streotomyces nodosus MH190-16F3 (FERM P No.

7985) is used.

**3.** The process as defined in claims 1 and 2 wherein the cultivation is carried through in a liquid submerged culture medium comprising nutritional carbon sources and nitrogen sources and optionally inorganic salts and/or antifoaming agents at a temperature of from 20 to 35° C and a cultivation time of from 2 to 4 days, the produced compounds are recovered from the culture broth by extraction with an organic waterimmiscible solvent and from the mycel with an aliphatic ester or ketone, a lower alkanol, or chloroform, and are recovered from the extracts, separated and purified by subjecting the extracts to column or liquid chromatography on a suitable adsorbent.

**Revendications**

**Revendications pour les Etats contractants suivants: BE, CH, DE, FR, GB, IT, LI, LU, NL, SE**

**1.** Composé de formule I

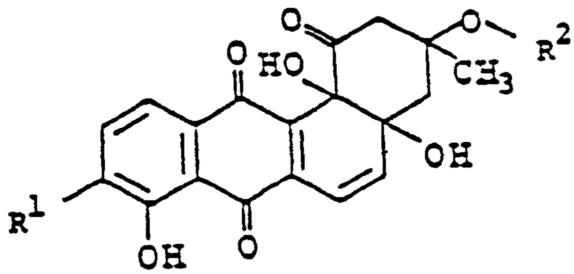

(I)

dans laquelle $R^1$ représente l'un des radicaux de formules suivantes:

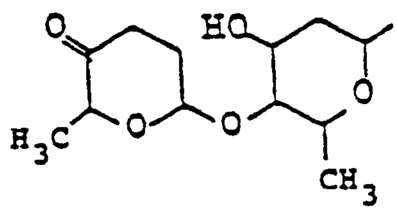

II

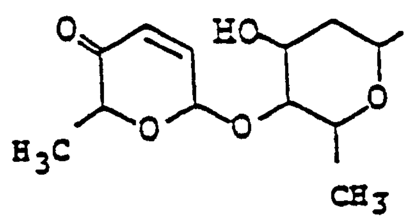

III

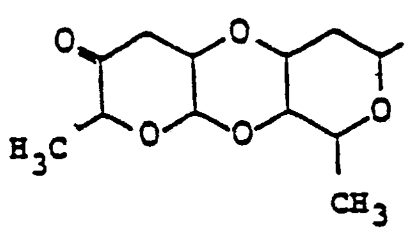

IV

et $R^2$ représente un atome d'hydrogène ou l'un des radicaux de formules:

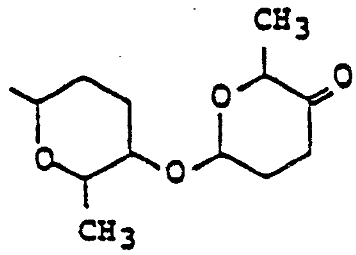

V

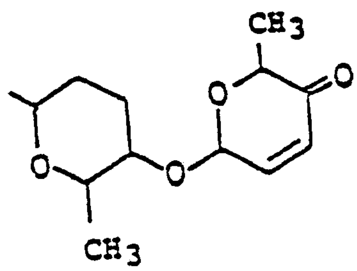

VI

2. Composé selon la revendication 1, dans lequel $R^1$ est le radical de formule III et $R^2$ est le radical de formule VI.

3. Composé selon la revendication 1, dans lequel $R^1$ est le radical de formule IV et $R^2$ est le radical de formule VI.

4. Composé selon la revendication 1, dans lequel $R^1$ est le radical de formule II et $R^2$ est le radical de formule V.

5. Composé selon la revendication 1, dans lequel $R^1$ est le radical de formule IV et $R^2$ est le radical de formule V.

6. Composé selon la revendication 1, dans lequel $R^1$ est le radical de formule III et $R^2$ est un atome d'hydrogène.

7. Composé selon la revendication 1, dans lequel $R^1$ est le radical de formule IV et $R^2$ est un atome d'hydrogène.

8. Composé selon la revendication 1, dans lequel $R^1$ est le radical de formule II et $R^2$ est un atome d'hydrogène.

9. Composition pharmaceutique comprenant en tant que composant actif un composé de formule I tel que défini dans la revendication 1, en association avec un véhicule pharmaceutiquement acceptable.

10. Utilisation d'un composé de formule I tel que défini dans la revendication 1, pour la fabrication d'un médicament efficace contre des cellules tumorales polyrésistantes à des médicaments.

11. Souche de micro-organisme Streptomyces nodosus MH190-16F3 (FERM P n° 7985).

12. Procédé pour la préparation d'un composé de formule I

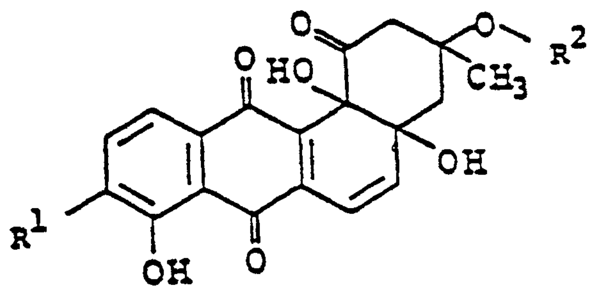

(I)

dans lequel $R^1$ représente l'un des radicaux de formules suivantes:

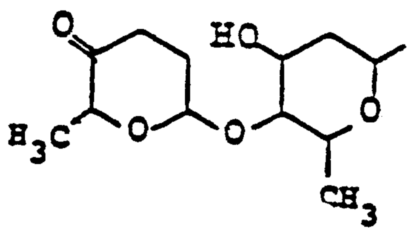

II

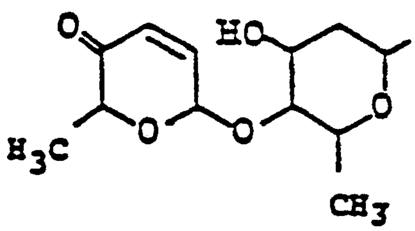

III

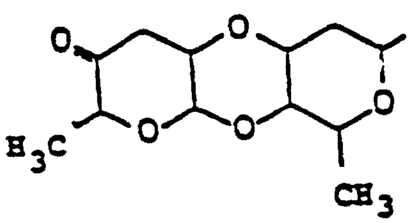

IV

et $R^2$ représente un atome d'hydrogène ou l'un des radicaux de formules:

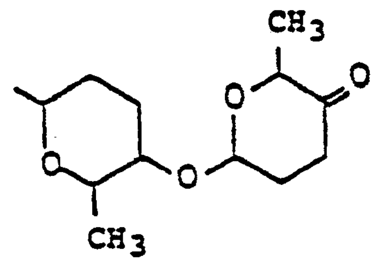

V

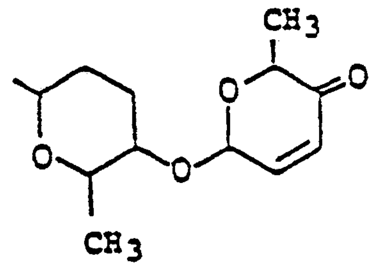

VI

comprenant la culture aérobie d'une souche de micro-organismes appartenant au genre Streptomyces, dans un milieu de culture approprié, et la récupération du produit à partir du bouillon de culture, et éventuellement à partir du mycélium, par des processus classiques d'extraction et/ou d'adsorption.

**13.** Procédé selon la revendication 12, dans lequel on utilise la souche Steptomyces nodosus MH190-16F3 (FERM P n° 7985).

**14.** Procédé selon les revendications 12 et 13, dans lequel la culture est effectuée dans un milieu de culture submergé comprenant des sources nutritives de carbone et des sources nutritives d'azote et éventuellement des sels minéraux et/ou des agents antimousse, à une température de 20 à 35 °C et pendant une durée de culture de 2 à 4 jours, les composés produits sont recueillis à partir du bouillon de culture par extraction avec un solvant organique non miscible à l'eau, et à partir du mycélium avec un ester aliphatique ou une cétone, un alcanol inférieur ou le chloroforme, et sont récupérés à partir des extraits, séparés et purifiés par exposition des extraits à une chromatographie liquide ou sur colonne, sur un adsorbant approprié.

**Revendications pour l'Etat contractant suivant: AT**

**1.** Procédé pour la préparation d'un composé de formule I

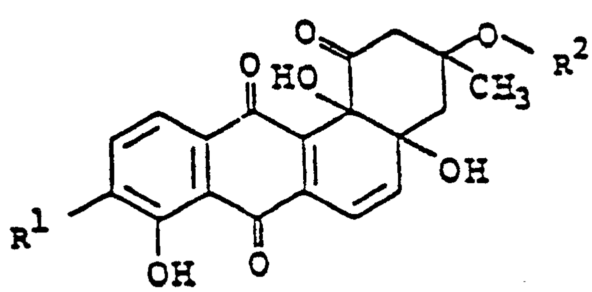

(I)

dans lequel $R^1$ représente l'un des radicaux de formules suivantes:

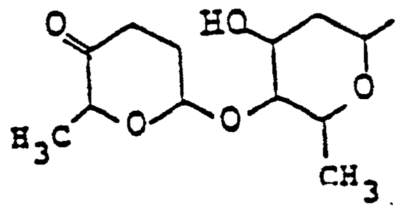

II

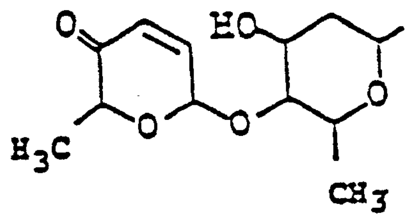

III

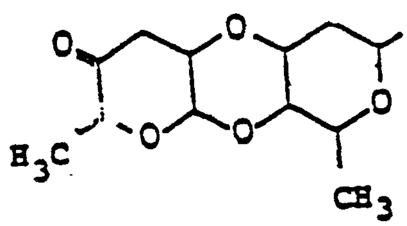

IV

et $R^2$ représente un atome d'hydrogène ou l'un des radicaux de formules:

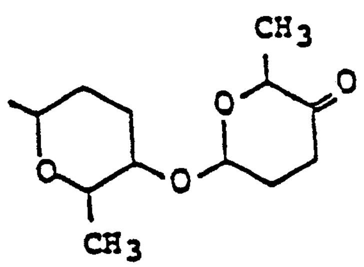

V

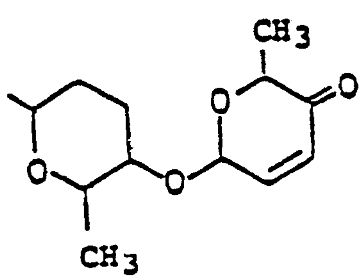

VI

comprenant la culture aérobie d'une souche de micro-organisme appartenant au genre Streptomyces, dans un milieu de culture approprié, et la récupération du produit à partir du bouillon de culture, et éventuellement à partir du mycélium, par des processus classiques d'extraction et/ou d'adsorption.

2. Procédé selon la revendication 1, dans lequel on utilise la souche Steptomyces nodosus MH190-16F3 (FERM P n° 7985).

3. Procédé selon les revendications 1 et 2, dans lequel la culture est effectuée dans un milieu de culture submergé comprenant des sources nutritives de carbone et des sources nutritives d'azote et éventuellement des sels minéraux et/ou des agents antimousse, à une température de 20 à 35° C et pendant une durée de culture de 2 à 4 jours, les composés produits sont recueillis à partir du bouillon de culture par extraction avec un solvant organique non miscible à l'eau, et à partir du mycélium avec un ester aliphatique ou une cétone, un alcanol inférieur ou le chloroforme, et sont récupérés à partir des extraits, séparés et purifiés par exposition des extraits à une chromatographie liquide ou sur colonne ou sur un adsorbant approprié.

**Patentansprüche**
**Patentansprüche für folgende Vertragsstaaten: BE, CH, DE, FR, GB, IT, LI, LU, NL, SE**

1. Eine Verbindung der Formel I

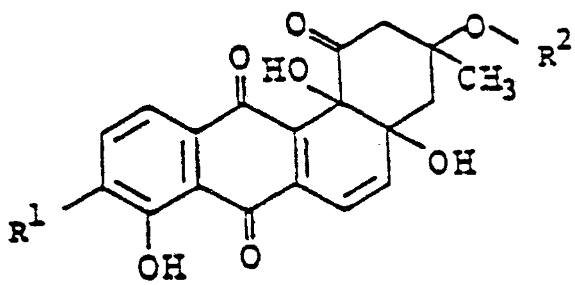

[I]

in welcher $R^1$ einen der Reste der folgenden Formeln

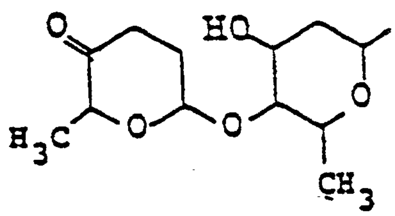

II

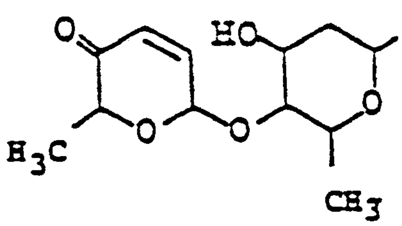

III oder

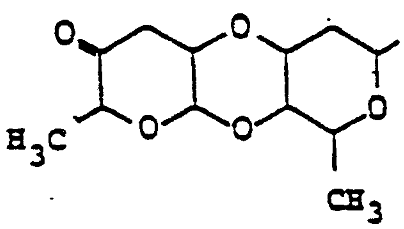

IV

bedeutet und $R^2$ Wasserstoff oder einen der Reste der Formeln

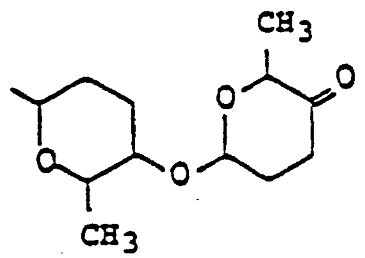

V oder

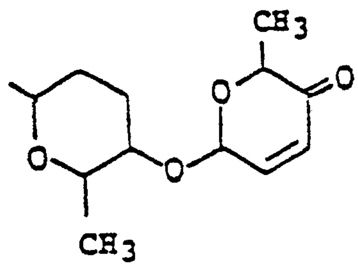

VI

bedeutet.

2. Verbindung wie in Anspruch 1 definiert, in welcher $R^1$ der Rest der Formel III und $R^2$ der Rest der Formel VI ist.

3. Verbindung wie in Anspruch 1 definiert, in welcher $R^1$ der Rest der Formel IV und $R^2$ der Rest der Formel VI ist.

4. Verbindung wie in Anspruch 1 definiert, in welcher $R^1$ der Rest der Formel II und $R^2$ der Rest der Formel V ist.

5. Verbindung wie in Anspruch 1 definiert, in welcher $R^1$ der Rest der Formel IV und $R^2$ der Rest der Formel V ist.

6. Verbindung wie in Anspruch 1 definiert, in welcher $R^1$ der Rest der Formel III und $R^2$ Wasserstoff ist.

7. Verbindung wie in Anspruch 1 definiert, in welcher $R^1$ der Rest der Formel IV und $R^2$ Wasserstoff ist.

8. Verbindung wie in Anspruch 1 definiert, in welcher $R^1$ der Rest der Formel II und $R^2$ Wasserstoff ist.

9. Pharmazeutische Zubereitung, welche als aktiven Bestandteil eine Verbindung der Formel I, wie in Anspruch 1 definiert, in Verbindung mit einem pharmazeutisch acceptablen Trägerstoff enthält.

10. Verwendung einer Verbindung der Formel 1, wie in Anspruch 1 definiert, für die Herstellung eines Arzneimittels welches gegen Tumorzellen wirksam ist, die gegenüber vielen pharmazeutischen Wirkstoffen resistent sind.

11. Der Mikroorganismusstamm Streptomyces nodosus MH190-16F3 (FERM P-No.7985).

12. Verfahren zur Herstellung einer Verbindung der Formel I

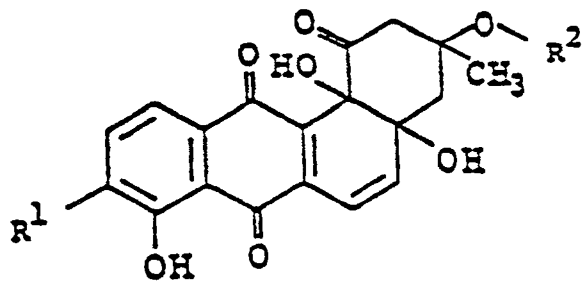

[I]

in welcher $R^1$ einen der Reste der folgenden Formeln

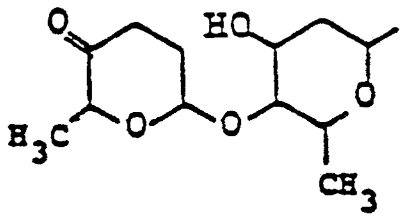

II

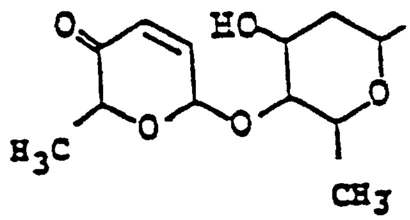

III oder

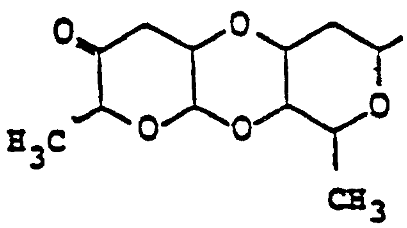

IV

bedeutet und $R^2$ Wasserstoff oder einen der Reste der Formeln

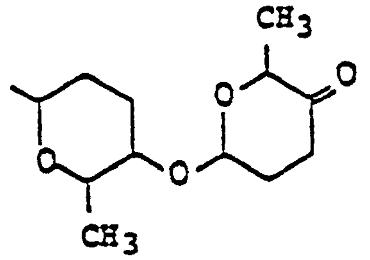

V oder

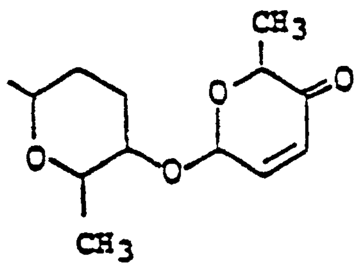

VI

bedeutet, dadurch gekennzeichnet, dass ein Mikroorganismusstamm des Typs Streptomyces in einem geeigneten Kulturmedium aerob gezüchtet wird und das Produkt aus der Kulturbrühe und gegebenenfalls aus dem Mycel durch übliche Extraktions- und/oder Adsorptionsverfahren wiedergewonnen wird.

**13.** Verfahren nach Anspruch 12, bei welchem der Stamm Streptomyces nodosus MH190-16F3 (FERM P-No.7985) verwendet wird.

**14.** Verfahren nach den Ansprüchen 12 und 13, bei welchem die Züchtung in einem flüssigen Submerskulturmedium, welches Kohlenstoff-Nährquellenund Stickstoffquellen und gegebenenfalls anorganische Salze und/oder Antischaummittel enthält, bei einer Temperatur von 20 bis 35 °C und einer Züchtungsdauer von 2 bis 4 Tagen durchgeführt wird, die hergestellten Verbindungen aus der Kulturbrühe durch Extraktion mit einem organischen, mit Wasser mischbaren Lösungsmittel, und aus dem Mycel mit einem aliphatischen Ester oder Keton, einem niedrigen Alkanol oder Chloroform gewonnen werden und aus den Extrakten wiedergewonnen, abgetrennt und gereinigt werden, indem die Extrakte einer Säulen- oder Flüssigchromatographie auf einem geeigneten Adsorbens unterworfen werden.

**Patentansprüche für folgenden Vertragsstaat: AT**

**1.** Verfahren zur Herstellung einer Verbindung der Formel I

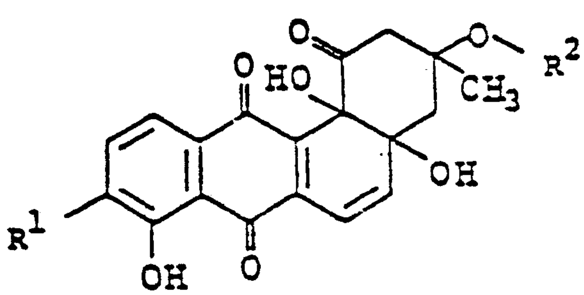

[I]

in welcher $R^1$ einen der Reste der folgenden Formeln

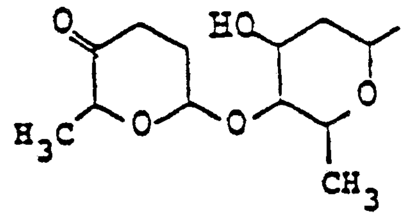

II

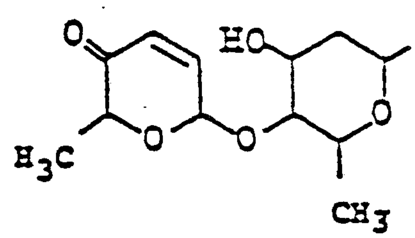

III oder

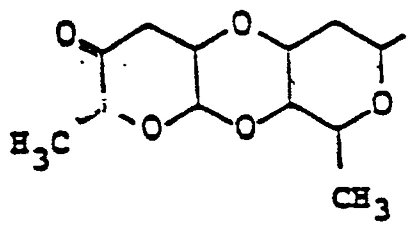

IV

bedeutet und $R^2$ Wasserstoff oder einen der Reste der Formeln

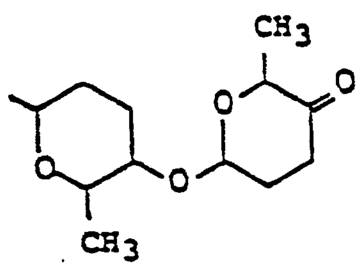

V oder

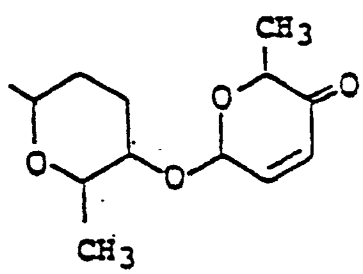

VI

bedeutet, dadurch gekennzeichnet, dass ein Mikroorganismusstamm des Typs Streptomyces in einem geeigneten Kulturmedium aerob gezüchtet wird und das Produkt aus der Kulturbrühe und gegebenenfalls aus dem Mycel durch übliche Extraktions- und/oder Adsorptionsverfahren wiedergewonnen wird.

**2.** Verfahren nach Anspruch 1, bei welchem der Stamm Streptomyces nodosus MH190-16F3 (FERM P.No. 7985) verwendet wird.

3. Verfahren nach den Ansprüchen 1 und 2, bei welchem die Züchtung in einem flüssigen Submerskulturmedium, welches Kohlenstoff-Nährquellenund Stickstoffquellen und gegebenenfalls anorganische Salze und/oder Antischaummittel enthält, bei einer Temperatur von 20 bis 35 °C und einer Züchtungsdauer von 2 bis 4 Tagen durchgeführt wird, die hergestellten Verbindungen aus der Kulturbrühe durch Extraktion mit einem organischen, mit Wasser mischbaren Lösungsmittel, und aus dem Mycel mit einem aliphatischen Ester oder Keton, einem niedrigen Alkanol oder Chloroform gewonnen werden und aus den Extrakten wiedergewonnen, abgetrennt und gereinigt werden, indem die Extrakte einer Säulen- oder Flüssigchromatographie auf einem geeigneten Adsorbens unterworfen werden.

FIG.1

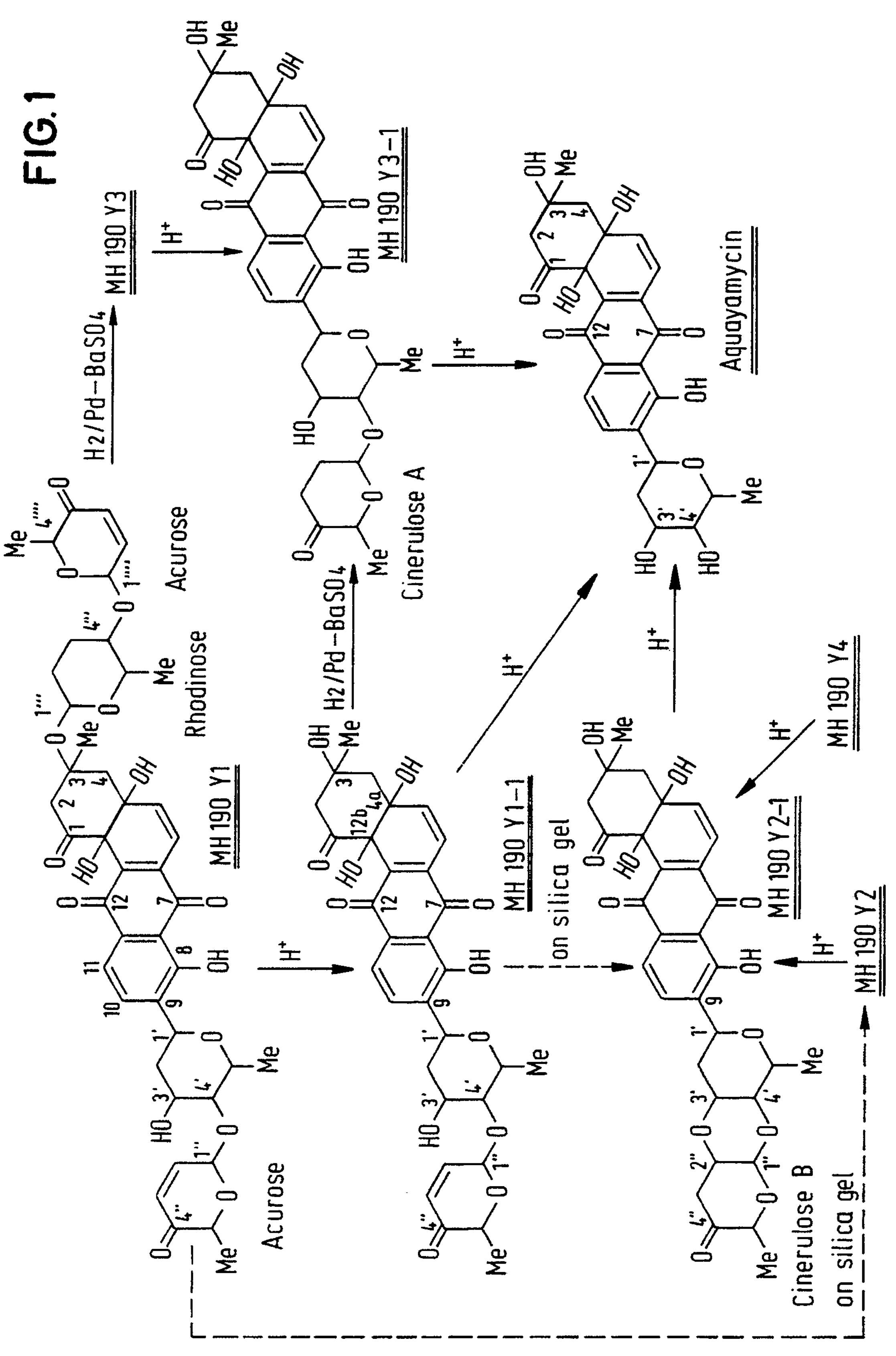


MH 190 Y1
Acurose
Rhodinose
Acurose
MH 190 Y3
MH 190 Y3-1
Cinerulose A
Aquayamycin
MH 190 Y1-1
MH 190 Y2-1
Cinerulose B
MH 190 Y4
MH 190 Y2

FIG. 2

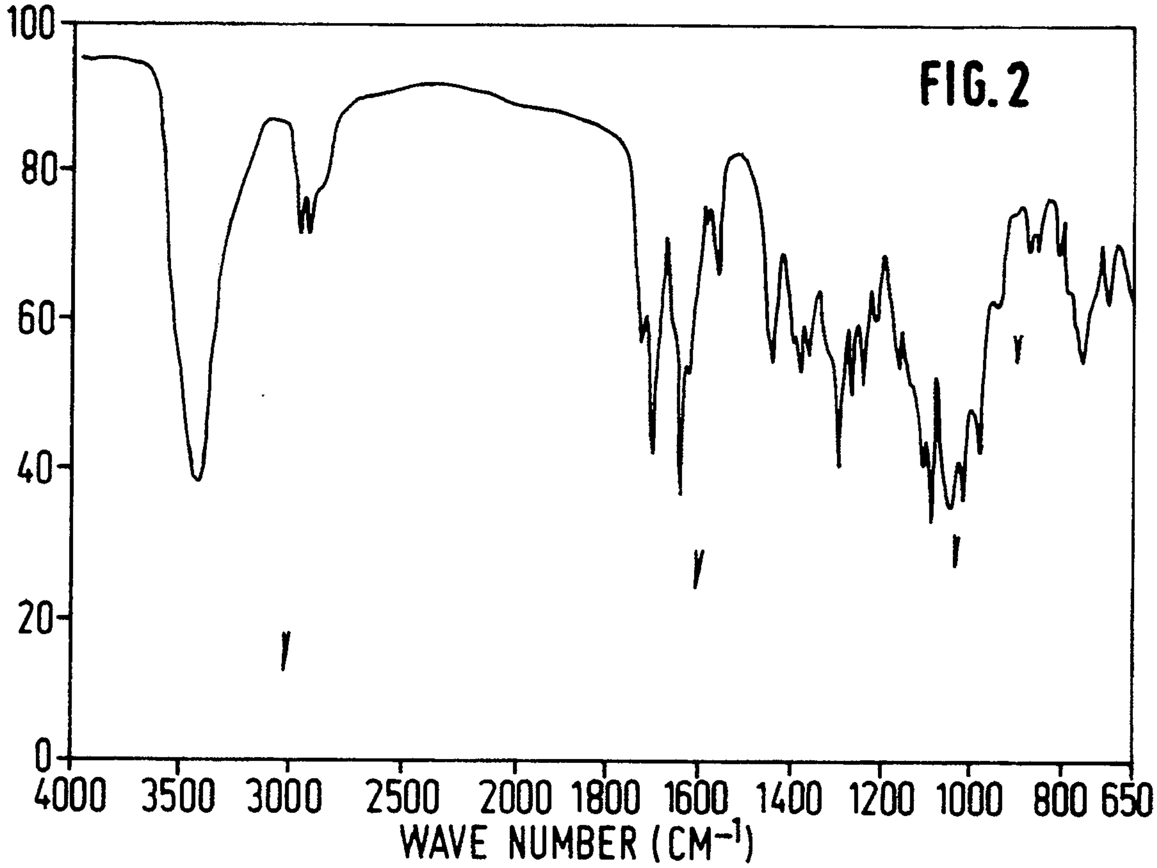
100
80
60
40
20
0
4000
3500
3000
2500
2000
1800
1600
1400
1200
1000
800
650
WAVE NUMBER (CM$^{-1}$)

FIG. 3

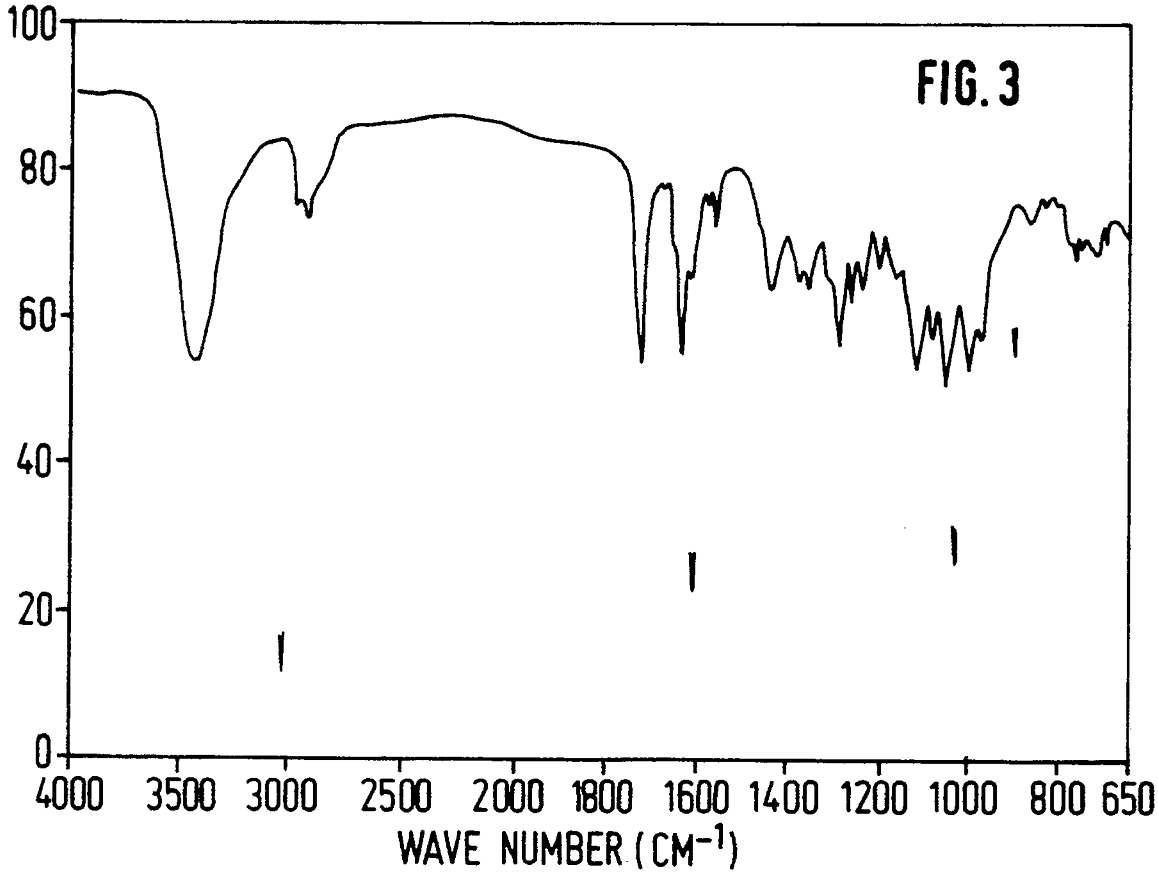
100
80
60
40
20
0
4000
3500
3000
2500
2000
1800
1600
1400
1200
1000
800
650
WAVE NUMBER (CM$^{-1}$)

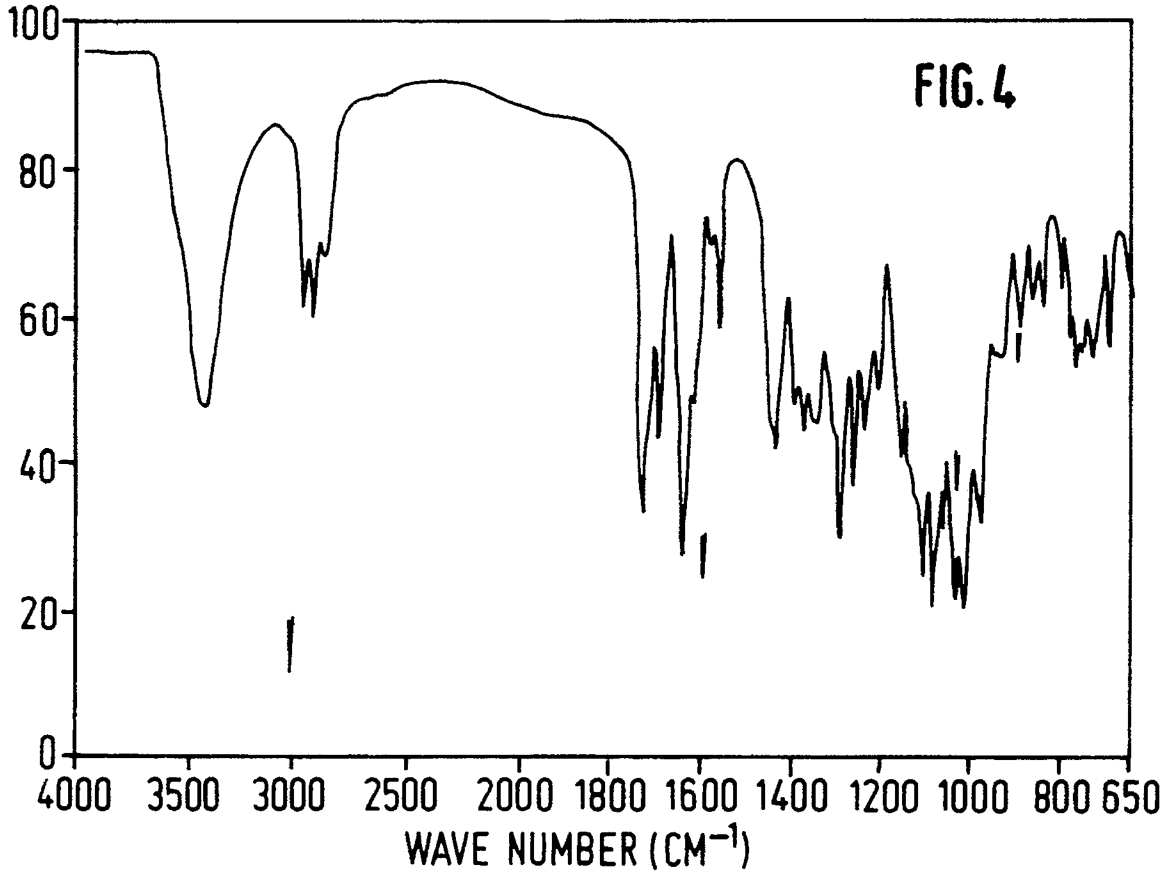
100
80
60
40
20
0
4000 3500 3000 2500 2000 1800 1600 1400 1200 1000 800 650
WAVE NUMBER (CM⁻¹)

FIG. 4

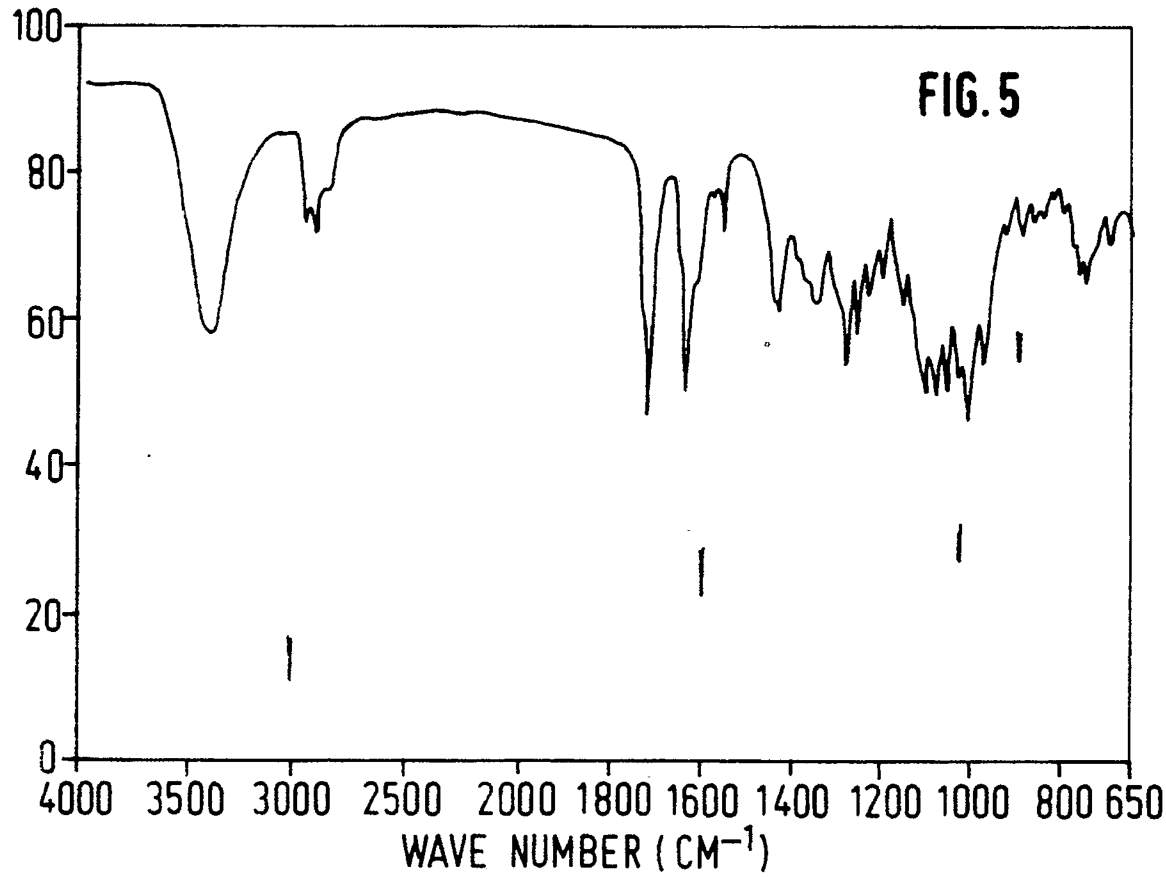
100
80
60
40
20
0
4000 3500 3000 2500 2000 1800 1600 1400 1200 1000 800 650
WAVE NUMBER (CM⁻¹)

FIG. 5

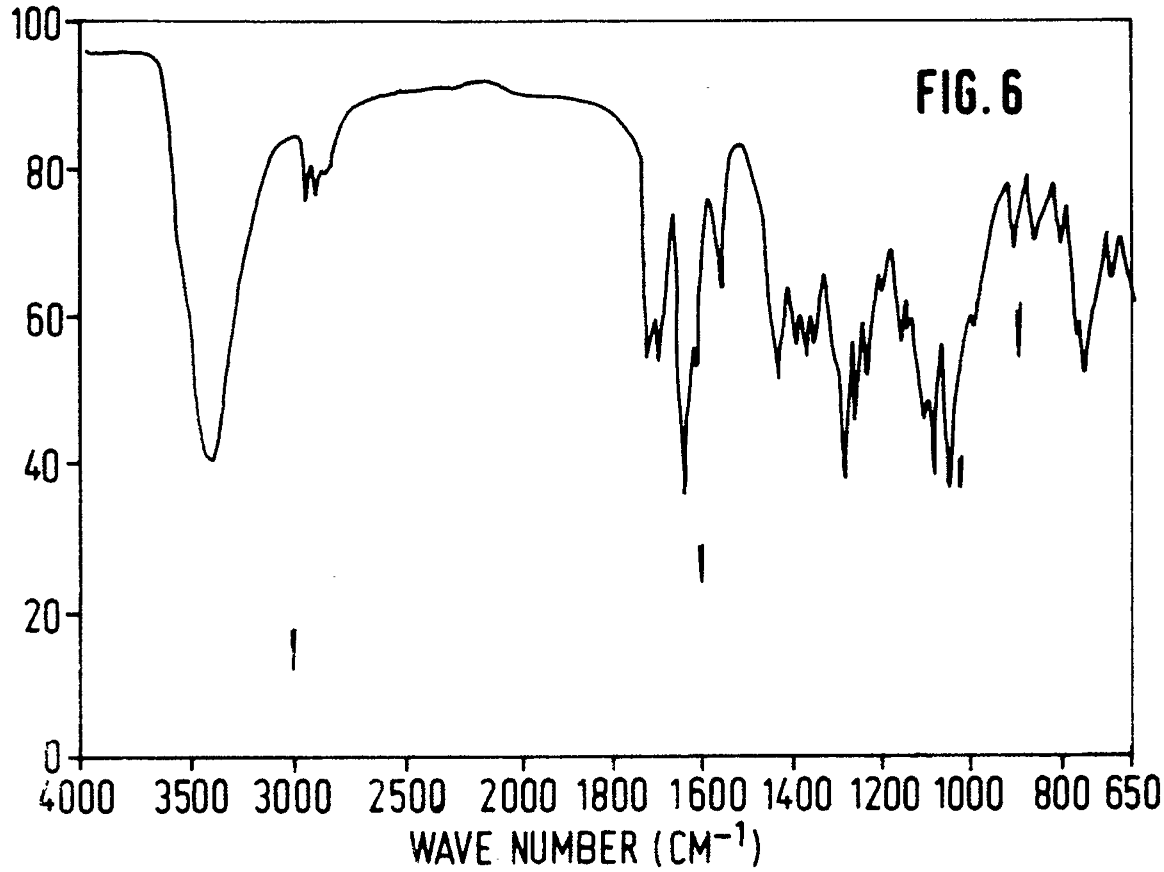
100
80
60
40
20
0
4000 3500 3000 2500 2000 1800 1600 1400 1200 1000 800 650
WAVE NUMBER (CM$^{-1}$)

FIG. 6

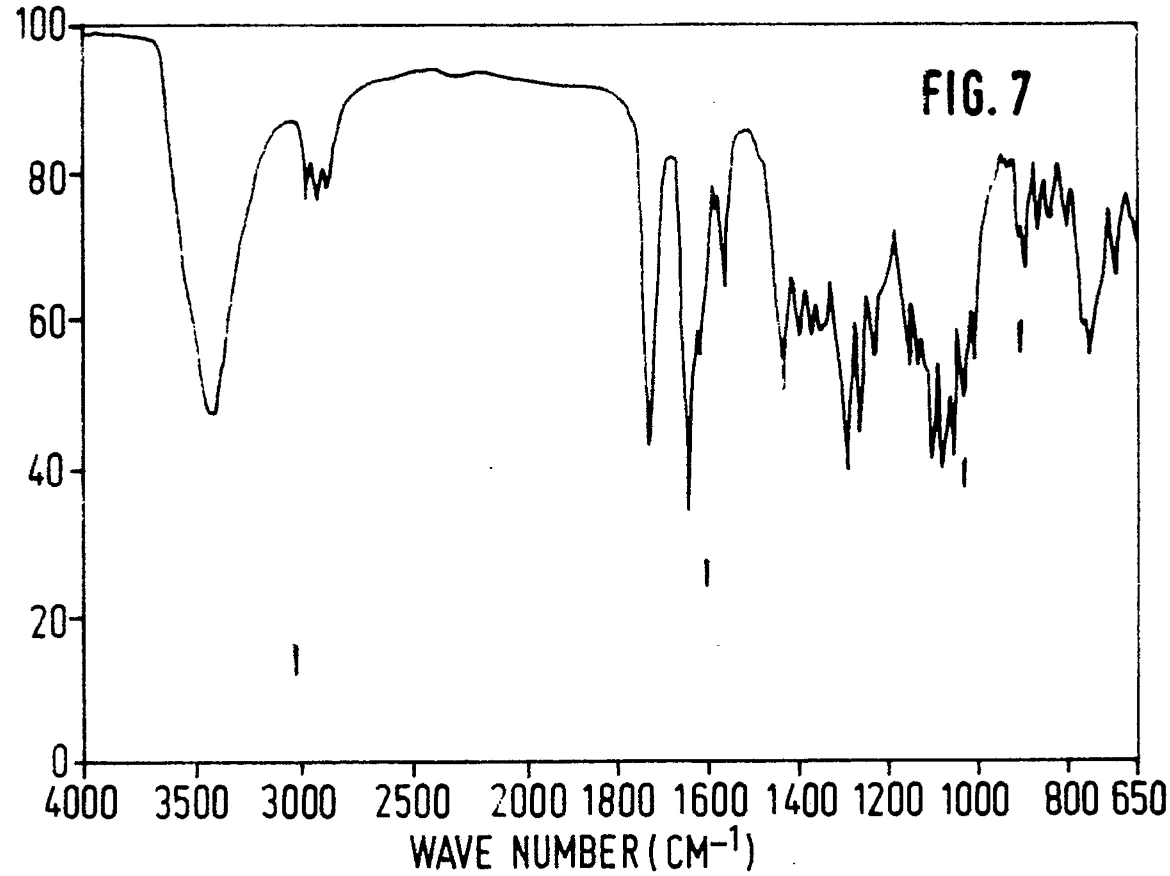
100
80
60
40
20
0
4000 3500 3000 2500 2000 1800 1600 1400 1200 1000 800 650
WAVE NUMBER (CM$^{-1}$)

FIG. 7

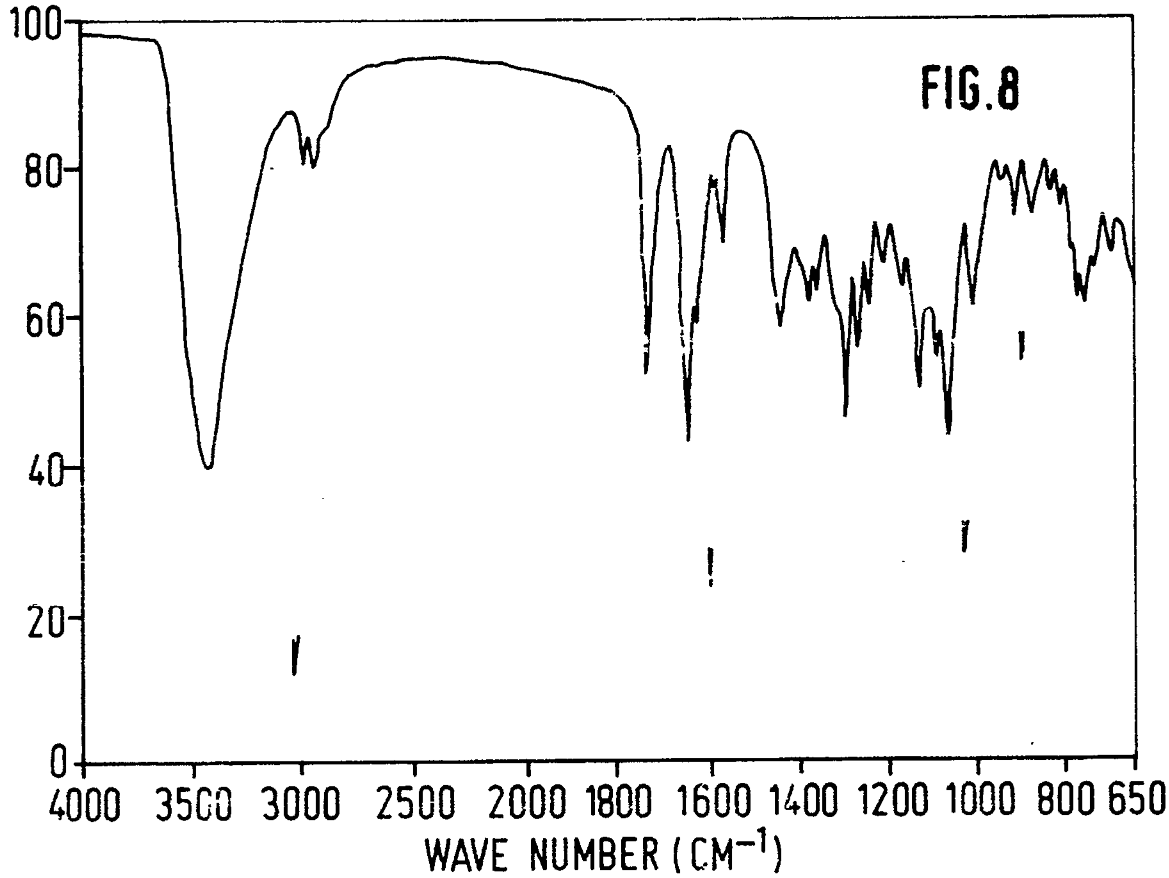
100
80
60
40
20
0
4000 3500 3000 2500 2000 1800 1600 1400 1200 1000 800 650
WAVE NUMBER (CM⁻¹)

FIG.8